# EUROPEAN PATENT SPECIFICATION

(11) **EP 0 507 224 B1**
(45) Date of publication and mention of the grant of the patent: **30.10.1996**
(21) Application number: 92105334.4
(22) Date of filing: 27.03.1992
(51) Int. Cl.: A61K 9/00, A61K 47/48

(54) **Use of combinations of gelling polysaccharides and finely divided drug carrier substrates in topical ophthalmic compositions**
Verwendung einer Kombination aus gelbindender Polysacchariden und fein verteilten Arzneimittelträgern in ophtalmischen topischen Zusammensetzungen
Utilisation d'une combinaison de polysaccharides gélifiants et de supports de principe actif finement divisés dans des compositions topiques ophtalmologiques

(30) Priority: 27.03.1991 US 676146
(43) Date of publication of application: 07.10.1992
(73) Proprietor: ALCON LABORATORIES, INC., Ft. Worth, TX 76134 (US)
(72) Inventor: Missel, Paul Joseph Tracy, Arlington, Texas 76016 (US); Jani, Rajni, Fort Worth, Texas 76109 (US); Lang, John C., Arlington, Texas 76017 (US)
(74) Representative: Reinhard - Skuhra - Weise & Partner

(56) References cited:
- EP-A- 0 424 043
- EP-A- 0 429 732
- US-A- 4 136 173
- US-A- 4 136 177
- US-A- 4 136 178
- US-A- 4 911 920
- DERWENT FILE SUPPLIER, 1989, AN=89-297785 [41], Derwent Publications Ltd, London, GB; & JP-A-1 221 306 (KYOEI KAGAKU KOGYO) 04-09-1989

## Description

### Field of the Invention

The present invention relates to sustained release topical ophthalmic compositions. In particular, this invention relates to a combination of drug, gelling polysaccharides and finely-divided drug carrier substrates in the compositions and their use for the manufacture of a medicament for the controlled administration of a drug to humans and animals wherein the compositions are administered as liquids which thicken to form gels upon instillation into the eye. Furthermore, the invention relates to a non-drug containing ophthalmic composition and the use thereof.

### Description of the Related Art

There have been a multiplicity of liquids, ointments, gels and inserts used as vehicles in topical ophthalmic formulations. Liquid compositions for drop-wise instillation to the eye provide for easy formulation, but do not provide an accurate dosage amount, as portions of the liquid are often blinked away during their administration. Ointments and gels, while providing more accurate administration, often interfere with patient vision. Ocular inserts, both bioerodible and non-bioerodible, are also available and allow for less frequent administration of drug; however, these inserts require complex and detailed preparation. An additional problem with the non-bioerodible inserts is that they must be removed after use.

U.S. Patents Nos. 4,136,173 (Pramoda et al), 4,136,177 (Lin et al.) and 4,136,178 (Lin et al) disclose the use of therapeutic compositions containing xanthan gum and locust bean gum which are delivered in liquid form and which gel upon instillation. In these three patents, the mechanism for transition from liquid to gel is due to a change in pH. U.S. Patent No. 4,861,760 (Mazuel et al) discloses ophthalmological compositions containing gellan gum which are administered as non-gelled liquids and which gel upon instillation due to the change in ionic strength.

Commonly assigned U.S. Patent Application Serial No. 07/641,214, filed on January 15, 1991, discloses ophthalmic formulations containing carrageenans and furcellarans (hereinafter collectively referred to as "carrageenans") which are administered as liquids or as partially gelled liquids which gel upon instillation into the eye.

Ph.D. thesis submitted to the University of Kentucky (Stalker, 1983) discloses the use of an ion-exchange resin as an improved ophthalmic drug delivery system. U.S. Patent No. 4,911,920 (Jani et al) discloses anti-glaucoma compositions containing combinations of ion-exchange resins and mucomimetic polymers.

### Summary of the Invention

The present invention is directed to topical ophthalmic compositions comprising combinations of gelling polysaccharides (defined below) and finely-divided drug carrier substrates (hereinafter "DCS" and defined below) to provide comfort and sustained release of drug to the eye, as well as their use. In addition, the compositions without drug can be administered in order to lubricate the eye or to supplement tears in the treatment of, for example, dry eye. The compositions are administered as liquids or partially gel led liquids (hereinafter collectively referred to as "liquids") which thicken to form gels upon instillation into the eye.

### Brief Description of the Drawing

Reference is now made to the drawings accompanying the application.

Figure 1 shows the results of in vitro, controlled release concentration versus time for several formulations tested (summarized in Table 1 and described in Example 29): a), b) concentration/time profiles; c), d) cumulative release profiles.

Figure 2, showing in vivo release of S-betaxolol from gelable formulations placed in rabbit cul-de-sacs, illustrates the consistency between the in vitro and in vivo measurements.

### Detailed Description of the Invention

As used herein, the term "gelling polysaccharide" means any polysaccharide capable of a reversible liquid-to-gel transition based on a change in ionic strength or pH. Such factors as a change in the temperature, amount and type of DCS, and characteristics and concentrations of drugs or other adjuvants may also affect the ability of the gelling polysaccharides to undergo a liquid-to-gel transition. Suitable gelling polysaccharides include, but are not limited to: xanthan gum, locust bean gum, gellan gum, carrageenans and combinations thereof. These gelling polysaccharides are discussed in detail in U.S. Patents Nos. 4,136,173, 4,136,177, 4,136,178, 4,861,760, and U.S. Patent Application Serial No. 07/641,214, respectively. The contents of these patents and patent applications relating to the gelling polysaccharides cited above are hereby incorporated by reference herein.

The preferred gelling polysaccharides of the present invention are the carrageenans, especially carrageenans with not more than 1.0 sulfate moiety per disaccharide unit such as eucheuma carrageenan and furcellaran. These provide both great contrast in the ratio of gel elastic modulus to viscosity over the temperature ranges of interest, as explained below, as well as limited response to drug counter-ions, especially as to their influence on gelation.

The DCS component of the present compositions is added to provide an additional means of controlling release, as well as to prevent the stinging which often occurs with the topical administration of certain drugs, such as S-betaxolol. As used herein, the term "finely-divided drug carrier substrate" (or "DCS") means finely-divided solids, colloidal particles, or soluble polymers and/or polyelectrolytes which are capable of selective adsorption or binding with drug molecules. Examples of DCS include, but are not limited to: finely-divided silica, such as fumed silica, silicates and bentonites; ion exchange resins, which can be anionic, cationic, zwitterionic or non-ionic, including combinations thereof; and soluble polymers such as alginic acid, pectin, soluble carrageenans, carbomers (e.g., Carbopole®) and polystyrene sulfonic acid. Preferred DCS are the ion exchange resins. Some resins which are used in chromatography make ideal DCS for binding drugs in the compositions of the present invention.

Ion exchange resins are typically in the form of beads which may be either porous or non-porous. Porous beads can be advantageous because they provide more surface area for increased drug binding and improved sustained release.

Functional groups which may be incorporated into DCS polymers include acids, bases or neutral hydrophobic or hydrophilic groups to influence the interaction with the drug. Specific functional groups may include, but are not limited to: sulfonic acid, carboxylic acid, phosphoric acid, aromatic groups such as phenyl or pyridinium, alkyl carbon chains, polyoxyethylene, polyoxypropylene, carboxymethyl, sulfopropyl, polyglycol and combinations thereof. The choice of functional group will depend on the drugs to be delivered, especially their charge at physiologic pH and that of the composition. For example, drugs with a positive charge at the desired composition pH will typically be formulated with resins having anionic functional groups at the composition pH.

Cationic exchange resins are characterized as either strongly acidic, such as those having sulfonic acid functionality, or weakly acidic, such as those having carboxylic acid functionality. Anionic exchange resins are characterized as either strongly basic, containing, for example, quaternary ammonium functionalities, or weakly basic, containing, for example, amines. Also disclosed are resins which may have any of a variety of functionalities whose charges offset each other (i.e., zwitterions), resulting in a neutral resin, or they may be comprised of nonionic polymers having any variety of hydrophilic functional groups.

The choice of resin functional group density (hereinafter "charge density") will also depend on the nature of the drug to be delivered. For example, drugs having multiple sites capable of binding or adhering to a resin (multiple resin binding sites) such as tobramycin, are strongly attracted to resins having relatively high charge densities, such as Amberlite®. Such combinations would not necessarily be desirable, since the resin-drug affinity would be so great that little or no drug would be available to the eye in a reasonable time period. Therefore, drugs having multiple resin binding sites are preferably combined with resins having relatively low charge densities, such as carboxymethyl Sephadex®. This will provide for a composition wherein the drug is available to the eye, but over a period of time. On the other hand, other drugs which do not have multiple resin binding sites are preferably combined with resins having a relatively high charge density in order to achieve a sustained release. Drug binding can be improved by converting resin salts to their acid/base forms.

The size of the DCS can be important, both with respect to mode of action and comfort. The average particle size of the typical commercially available form of the DCS material of choice, an ion exchange resin, is about 40 to about 150 microns. Such particles are most conveniently reduced to a particle size range of about 1.0 to about 25.0 microns, preferably between about 1.0 and 10.0 microns, by ball milling, according to known techniques. In the alternative, small particles may be synthesized in the optimal size range of 3-7 microns. Although this procedure can be more expensive, it is superior in providing a more uniform and narrow distribution of sizes in the preferred range.

The DCS component is present in the compositions of the present invention at a level in the range of about 0.05 to about 10.0% by weight. For particulate DCS, the average particle size diameter ranges from 1 to 20 microns. The amount of DCS and its characteristics (e.g., amount of cross-linking, particle size) may be varied in order to produce the desired time-release profile for the chosen drug. A long time-release profile, desirable for a drug having a short biological half-life, such as apraclonidine, may be achieved by using a large excess of DCS (i.e., the number of DCS binding/exchange sites is several times that of the drug(s) being delivered). An intermediate release profile, suggested for a drug such as pilocarpine, having a reasonably good half-life (4 hours), may be obtained by using a smaller excess of DCS (i.e., the number of DCS binding sites is roughly equivalent to that of the drug(s) being delivered). For drugs which have unpleasant side effects, such as S-betaxolol, a preferred release profile is usually a rapid initial release of an amount of drug effective to cross the therapeutic threshold, followed by a sustained release tail, to maintain the therapeutic effect and to reduce or eliminate side effects. This may be achieved by using an excess of drug (as compared to the number of DCS binding sites).

DCS materials which can be used in the composition of the present invention may include, but are not limited to: fumed silica, e.g., Cab-O-Sil® (Cabot Corporation, Tuscola, IL); any of several categories of clays, such as hectorite (Macaloid®, NL Chemicals, Hightstown, NJ), montmorillonite (Gelwhite®, Southern Clay Products, Gonzales, TX), bentonite (e.g., refined bentonite such as Polargel®, American Colloid Company, Arlington Heights, IL), MAS (magnesium aluminum silicates, mixtures of bentonites and hectorites such as Veegum®, R.T. Vanderbilt Company, Norwalk, CT), organophilic clays (Claytone®, Southern Clay Products, Gonzales, TX); polystyrene/divinylbenzene, such as Amberlite IRP-69® and Duolite AP-143® (Rohm & Haas, Philadelphia, Pennsylvania) and RCX-20® (Hamilton, Reno, NV); polymethacrylic acid, such as Amberlite IRP-64 (Rohm & Haas); hydroxymethylmethacrylate (HEMA), such as HEMA-IEC BIO 1000 SB (Alltech Associates, Deerfield, Illinois); cross-linked dextran, such as Sephadex® (Dow Chemicals, Midland, Michigan); and alginic acid.

The compositions of the present invention may be formulated in many ways, for example, 1) a liquid formulation, wherein the composition is a low viscosity liquid which becomes a high viscosity liquid or a gel upon instillation in the eye; 2) a stiff gel formulation, wherein the composition is a weak gel which becomes a stiffer gel in the eye; and 3) a thixotropic formulation, wherein the composition is a viscous liquid when shaken, a weak gel when left standing for a period of time and which becomes a stiffer gel in the eye.

The different types of formulations discussed above exhibit different physical characteristics. For the sake of clarity and for ease of reference in the discussion below, "pre-dosed" refers to a formulation's characteristics before topical administration to the eye and "post-dosed" refers to a formulation's characteristics after administration into the eye.

The liquid formulations have a pre-dosed viscosity in the range of about 1 to 500 mPa·s (centipoise (cps)), with about 1 to about 200 mPa·s (cps) preferred, and about 1 to about 100 mPa·s (cps) most preferred. If the liquid formulations do not form a gel in the eye, but simply become more viscous, the post-dosed viscosity will be greater than about 50 mPa·s (cps), preferably greater than about 150 mPa·s (cps) and most preferably greater than about 300 mPa·s (cps). If the liquid formulations do form a gel in the eye, the gel will have a modulus of elasticity (Young's modulus) in the range of about 1 X 10⁴ to about 5 X 10⁵ dPa (dynes/cm²), with about 2 X 10⁴ to about 5 X 10⁵ dPa (dynes/cm²) preferred, and about 5 X 10⁴ to about 5 X 10⁵ dPa (dynes/cm²) most preferred. Gel strengths can be method-dependent; the method used was adapted from that of Lecacheux et al., Carbohydrate Polymers, 8:119-130 (1988). Post-dose gels are materials to which salts were added at physiologic levels.

The stiff gel formulations have a pre-dosed modulus of elasticity in the range of about 1 X 10⁴ to about 3 X 10⁵ dPa (dynes/cm²), with about 2 X 10⁴ to about 2 X 10⁵ dPa (dynes/cm²) preferred and about 5 X 10⁴ to about 1 X 10⁵ dPa (dynes/cm²), most preferred. The post-dosed stiff formulations are gels and will have a modulus of elasticity in the range of about 1 X 10⁴ to about 2 X 10⁶ dPa (dynes/cm²), with a 1 X 10⁵ to about 7 X 10⁵ dPa (dynes/cm²) preferred about 2 X 10⁵ to about 5 X 10⁵ dPa (dynes/cm²) most preferred.

The thixotropic formulations, when shaken, have a pre-dosed viscosity in the range of about 1 to about 5000 mPa·s (cps), with about 50 to about 1000 (cps) mPa·s preferred and about 200 to about 500 mPa·s (cps) most preferred. The pre-dosed gel forms of the thixotropic formulations have a modulus of elasticity in the range of about 1 X 10⁴ to about 2 X 10⁵ dPa (dynes/cm²), with about 2 X 10⁴ to about 1 X 10⁵ dPa (dynes/cm²) preferred and about 3 X 10⁴ to about 7 X 10⁴ dPa (dynes/cm²) most preferred. The post-dosed gels will have a modulus of elasticity in the range of about 1 X 10⁴ to about 2 X 10⁶ dPa (dynes/cm²), with about 2 X 10⁴ to about 1 X 10⁵ dPa (dynes/cm²) preferred and about 3 X 10⁴ to about 7 X 10⁴ dPa (dynes/cm²) most preferred.

Suitable ophthalmic agents ("drugs") which may be included in the compositions of the present invention and administered via the method of the present invention include, but are not limited to, the racemic and enantiomeric forms of following types of drugs:
- glaucoma agents, such as: β-blockers (e.g., betaxolol, timolol, and carteolol); α-agonists (e.g., apraclonidine and related 2-substituted amino imidazolines); carbonic anhydrase inhibitors (e.g., (+)-4-ethylamino-2,3-dihydro-4-H-2-methylthieno[3,2-e]-1,2-thiazine-6-sulfonamide-1,1-dioxide (ALO4414) and related aryl and heteroaryl sulfonamides); dopamine agonists and antagonists; miotic cholinergics (e.g., pilocarpine and carbachol); prostaglandins; ACE inhibitors; steroids (e.g., glucocorticoids and angiostatic steroids); and calcium channel blockers;
- anti-hypertensives;
- non-steroidal and steroidal anti-inflammatory agents, such as diclofenac and ketorolac, fluoromethalone acetate, prednisolone acetate, tetrahydrocortisol;
- anti-bacterials and anti-infectives, such as aminoglycosides (e.g., tobramycin); quinolones (e.g., ciprofloxacin and ofloxacin); beta-lactams (e.g., cephalosporins such as cefamandole);
- anti-fungals, such as natamycin;
- anti-virals, such as acyclovir and ganciclovir;
- anti-cataract agents and anti-oxidants;
- anti-allergics;
- growth factors such as EGF, FGF, PGDF; and
- prodrugs of the drug classes listed above.
Combinations of ophthalmic agents may also be used in the compositions of the present invention. Further, in formulations without ophthalmic agents, the present invention may also serve to supplement tears in the prevention or treatment of dry eye.

The compositions of the present invention may additionally include other ophthalmically acceptable components: for example, buffers (e.g., phosphate, borate and citrate), preservatives, and tonicity agents (e.g., sodium chloride and mannitol).

In general, for water-soluble drugs, the compositions of the present invention are formulated such that the DCS is added to the solution prior to the addition of drug (if any) and the gelling polysaccharide is added last, after all the other ingredients have been mixed. Where the drug to be included in the compositions of the present invention has a low solubility, it is preferred that the drug be added last; that is, after the addition of the gelling polysaccharide. In certain cases, it may also be preferred that the drug be separately sterilized (e.g., with radiation) and aseptically added to the other ingredients, which have been autoclaved according to the sterilization procedure described below.

Sterilization of the compositions can be accomplished by autoclaving. It is well known that an order of magnitude reduction in sterilization time is achieved for every 10°C rise in sterilization temperature. As the gelling polysaccharides tend to decompose and caramelize when heated, sterilization at higher temperatures with lower sterilization time is generally preferred. The preferred temperature range is greater than about 130°C, with a sterilization time of less than about 3 minutes when the pH of the composition is more than about 6. In the alternative, aseptic combinations of drug and gelling polysaccharide can be utilized when the hydration of the resin results in chemical instability of the drug or of the drug/DCS complex. In those instances where the final pH of the composition is less than 6, it is preferred that sterilization take place at a pH close to 7.4, then to adjust the pH by aseptic means to its final value.

In the compositions of the invention, the concentration of pharmaceutical active agent (drug) will range from about 0.01 parts per million (1 X 10⁻⁸) up to 50,000 parts per million, based on the total weight of the composition. The active components of the composition are contained in an ophthalmic vehicle as described herein.

The following Examples are presented to illustrate various aspects of the present invention: Examples 1-10 represent stiff gel formulations; Examples 11-20 represent thixotropic gel formulations; Examples 21-28 represent liquid formulations; and Example 29 contains a quantitative comparison of the release characteristics of various formulations. Amounts are given as percent by weight in volume (%w/v) unless otherwise indicated.

### Example 1

The following procedure was utilized to prepare a 50 milliliter (ml) batch of S-betaxolol formulation.

| INGREDIENT | AMOUNT (%w/v) |
|---|---|
| Eucheuma Carrageenan | 2.0 |
| S-betaxolol | 0.5 |
| Na₂HPO₄ | 0.1 |
| Mannitol | 3.5 |
| Amberlite® IRP-69 | 5.0 |
| NaOH or HCl | q.s. to pH 7.4 |
| Purified Water | q.s. to 100% |

Approximately 30 ml of water (about 2/3 of the final volume), 51 mg of Na₂HPO₄ (0.1 %w/v) and 1.75 g of mannitol (3.5 %w/v) were added to a beaker equipped with a magnetic stir bar. The mixture was stirred until the ingredients were dissolved, then 8.4 g of rinsed Acid Amberlite® IRP-69 (corresponding to 2.5 g dry weight) was added and the mixture stirred for another 15 minutes (min), until the Amberlite® IRP-69 was uniformly mixed; that is, until there were no lumps. The pH of the mixture was raised from 2 to 2.5 by the addition of 10 N NAOH. After the pH adjustment, 0.24 g of S-betaxolol (base) was added and the mixture stirred approximately 1/2 hour without adjusting the pH. The pH of the mixture was then adjusted to 3.3 by addition of 1 N NAOH. The mixture was stirred overnight (at least 12 hours) to ensure that the S-betaxolol was adequately bound to the Acid Amberlite® IRP-69. The pH of the mixture was then raised to 7.4 with 10 N NaOH and purified water added to bring the final volume to 45 ml. The mixture was then heated to 75°C and 1.0 g of eucheuma carrageenan (2 %w/v) added. (Two percent of one lot of eucheuma was adequate to provide a stiff gel as defined above; however, the amount may vary depending on the particular lot of carrageenan.) The mixture was then stirred, heated to 90°C and the temperature maintained for a half hour. After removing the mixture from the heat, water was added to bring the final volume to 50 ml and the osmolality was checked. The final osmolality was 308 milliOsmoles per kg (mOsm/kg).

The mixture was sterilized in an autoclave at 130°C for 3 minutes in containers having radii no greater than 1 centimeter (cm). After sterilization, the containers were removed from the autoclave and air cooled to room temperature or, alternatively, the containers were quench-cooled to below 50°C and air cooled to room temperature.

### Example 2

The following procedure was utilized to prepare a 50 ml batch of apraclonidine formulation.

| INGREDIENT | AMOUNT (%w/v) |
|---|---|
| Eucheuma Carrageenan | 2.0 |
| Apraclonidine | 1.0 |
| Na₂HPO₄ | 0.1 |
| Mannitol | 3.5 |
| Amberlite® IRP-69 | 3.0 |
| NaOH or HCl | q.s. to pH 7.4 |
| Purified Water | q.s. to 100% |

Water, Na₂HPO₄, mannitol, and 1.5 g of Acid Amberlite® IRP-69 were added as in Example 1 and pH adjusted as described. Apraclonidine (base), 1.0 %w/v, was added and the mixture stirred approximately 1/2 hour without adjusting pH. The pH of the mixture was then adjusted to 3 by addition of 1.0 N NaOH. The subsequent treatment of this mixture was the same as that described in Example 1.

### Example 3

The following procedure was utilized to prepare a 50 ml batch of pilocarpine formulation.

| INGREDIENT | AMOUNT (%w/v) |
|---|---|
| Eucheuma Carrageenan | 2.0 |
| Pilocarpine | 1.0 |
| Na₂HPO₄ | 0.1 |
| Mannitol | 3.5 |
| RCX-20® | 5.0 |
| NaOH or HCl | q.s. to pH 7.4 |
| Purified Water | q.s. to 100% |

Water, Na₂HPO₄, mannitol, and 2.5 g of acid RCX-20® were added as in Example 1 and pH adjusted as described. Pilocarpine (base), 1.0 %w/v, was added and the mixture stirred approximately 1/2 hour without adjusting pH. The pH of the mixture was then adjusted to 3 by addition of 1.0 N NaOH. The subsequent treatment of this mixture was the same as that described in Example 1.

### Example 4

The following procedure was utilized to prepare a 50 ml batch of tobramycin formulation.

| INGREDIENT | AMOUNT (%w/v) |
|---|---|
| Eucheuma Carrageenan | 2.0 |
| Tobramycin | 2.0 |
| Cellulose Phosphate | q.s. to pH 7.4 (5.2) |
| Purified Water | q.s. to 100% |

To a screwcap flask was added 1 g of tobramycin and 30 ml of deionized water. After stirring and dissolution, the pH was about 10. Sufficient (typically, 2.6 g) cellulose phosphate (acid) was added to reduce the pH to 7.4. The eucheuma, 1.0 g, was added while stirring and then water added to bring the final volume to 50 ml. The capped flask was heated to 90°C and stirred for 30 minutes. Upon cooling, the pH was no lower than 7.

### Example 5

The following procedure was utilized to prepare a 50 ml batch of tobramycin formulation.

| INGREDIENT | AMOUNT (%w/v) |
|---|---|
| Eucheuma Carrageenan | 2.0 |
| Tobramycin | 2.0 |
| Carboxymethyl Sephadex® | q.s. to pH 7.4 (4.0) |
| Purified Water | q.s. to 100% |

To a screwcap flask was added 1 g of tobramycin and 30 ml of deionized water. After stirring and dissolution, the pH was about 10. Sufficient (typically, 2 g) Carboxymethyl Sephadex® (acid) was added to reduce the pH to 7.4. The eucheuma, 1.0 g, was added while stirring and then water added to bring the final volume to 50 ml. The capped flask was heated to 90°C and stirred for 30 minutes. Upon cooling, the pH was no lower than 7.

### Example 6

The following procedure was utilized to prepare a 50 ml batch of tobramycin formulation.

| INGREDIENT | AMOUNT (%w/v) |
|---|---|
| Eucheuma Carrageenan | 2.0 |
| Tobramycin | 2.0 |
| Sulfopropyl Sephadex® | q.s. to pH 7.4 (7.2) |
| Purified Water | q.s. to 100% |

To a screwcap flask was added 1 g of tobramycin and 30 ml of deionized water. After stirring and dissolution, the pH was about 10. Sufficient (typically, 3.6 g) Sulfopropyl Sephadex® (acid) was added to reduce the pH to 7.4. The eucheuma, 1.0 g, was added while stirring and then water added to bring the final volume to 50 ml. The capped flask was heated to 90°C and stirred for 30 minutes. Upon cooling, the pH was no lower than 7.

### Example 7

The following procedure was utilized to prepare a 50 ml batch of tobramycin formulation.

| INGREDIENT | AMOUNT (%w/v) |
|---|---|
| Eucheuma Carrageenan | 2.0 |
| Tobramycin | 2.0 |
| Na₂HPO₄ | 0.06 |
| KH₂PO₄ | 0.03 |
| Amberlite® IRP-64 | q.s. to pH 7.4 (3.0) |
| Purified Water | q.s. to 100% |

To a screwcap flask were added 1 g of tobramycin, 32 mg dibasic sodium phosphate, 14 mg of monobasic potassium phosphate, and 1 g of ball-milled Amberlite® IRP-64, followed by 30 ml of deionized water. The suspension was equilibrated for 16 hours, during which time the pH dropped to 8.44. An additional 0.5 g of resin was added and the suspension equilibrated for another hour, at which time the pH was 7. Eucheuma carrageenan, 1 g, was added while the suspension was stirred and then water was added to bring the final volume to 50 ml. The capped flask was heated to 90°C and stirred for 30 minutes. Upon cooling, the pH was no lower than 7.

### Example 8

The following procedure was utilized to prepare a 50 ml batch of diclofenac formulation.

| INGREDIENT | AMOUNT (%w/v) |
|---|---|
| Eucheuma Carrageenan | 2.0 |
| Diclofenac | 1.0 |
| Na₂HPO₄ | 0.1 |
| Mannitol | 3.5 |
| Duolite® AP-143 | 5.0 |
| NaOH or HCl | q.s. to pH 7.4 |
| Purified Water | q.s. to 100% |

Water (30 ml), Na₂HPO₄, mannitol, and 2.5 g of Duolite AP 143® base were added to a flask. Diclofenac (acid), 1.0 %w/v, was added and the mixture stirred 36 hours without adjusting pH. The pH of the mixture was then adjusted to 7.4 by addition of 1.0 N HCl, and water was added to bring the final volume to 45 ml. The subsequent treatment of this mixture was the same as that described in Example 1.

### Example 9

The following procedure was utilized to prepare a 50 ml batch of S-betaxolol formulation.

| INGREDIENT | AMOUNT (%w/v) |
|---|---|
| Eucheuma Carrageenan | 2.0 |
| S-betaxolol HCl | 0.56 |
| Mannitol | 3.5 |
| Bentonite | 3.0 |
| NaOH or HCl | q.s. to pH 7.4 |
| Purified Water | q.s. to 100% |

Water, mannitol, 1.5 g of Bentonite and 0.28 g of S-betaxolol HCl were added as in Example 1, and pH adjusted to 7.4. The subsequent treatment of this mixture was the same as that described in Example 1.

### Example 10

The following procedure was utilized to prepare a 50 ml batch of S-betaxolol formulation.

| INGREDIENT | AMOUNT (%w/v) |
|---|---|
| Furcellaran | 2.0 |
| S-betaxolol | 0.5 |
| Na₂HPO₄ | 0.1 |
| Mannitol | 3.5 |
| Amberlite® IRP-69 | 5.0 |
| NaOH or HCl | q.s. to pH 7.4 |
| Purified Water | q.s. to 100% |

Water, Na₂HPO₄, mannitol, and 2.5 g of Amberlite® IRP-69 were added as in Example 1, and pH adjusted as described. S-betaxolol (base), 0.5%w/v, was added and the mixture stirred approximately 1/2 hour without adjusting pH. The pH of the mixture was then adjusted to 3 by addition of 1.0 N NaOH. The subsequent treatment of this mixture was the same as that described in Example 1, except that furcellaran was used in place of eucheuma.

### Example 11

The following procedure was utilized to prepare a 50 ml batch of S-betaxolol formulation.

| INGREDIENT | AMOUNT (%w/v) |
|---|---|
| Gelrite®* | 0.6 |
| S-betaxolol | 0.5 |
| Na₂HPO₄ | 0.1 |
| Mannitol | 3.5 |
| Amberlite® IRP-69 | 5.0 |
| NaOH or HCl | q.s. to pH 7.4 |
| Purified Water | q.s. to 100% |

| | |
|---|---|
| *Gelrite® is available from Kelco (San Diego, CA) and consists of gellan gum and some ions. | |

Water, Na₂HPO₄, mannitol, and 2.5 g of Acid Amberlite® IRP-69 were added as in Example 1 and pH adjusted as described. S-betaxolol (base), 0.5 %w/v, was added and the mixture stirred approximately 1/2 hour without adjusting pH. The pH of the mixture was then adjusted to 3 by addition of 1.0 N NaOH. The subsequent treatment of this mixture was the same as that described in Example 1, with the exception that 1 g of eucheuma was substituted by 0.3 g of Gelrite®.

### Example 12

The following procedure was utilized to prepare a 50 ml batch of tobramycin formulation.

| INGREDIENT | AMOUNT (%w/v) |
|---|---|
| Eucheuma Carrageenan | 1.2 |
| Pilocarpine | 1.0 |
| Na₂HPO₄ | 0.1 |
| Mannitol | 3.5 |
| RCX-20® | 5.0 |
| NaOH or HCl | q.s. to pH 7.4 |
| Purified Water | q.s. to 100% |

Water, Na₂HPO₄, mannitol, and 2.5 g of RCX-20® were added as in Example 1 and pH adjusted as described. Pilocarpine (base), 1%w/v, was added and the mixture stirred approximately 1/2 hour without adjusting pH. The pH of the mixture was then adjusted to 3 by addition of 1.0 N NaOH. The subsequent treatment of this mixture was the same as that described in Example 1, but with the addition of 0.6 g of eucheuma rather than 1.0 g.

### Example 13

The following procedure was utilized to prepare a 50 ml batch of tobramycin formulation.

| INGREDIENT | AMOUNT (%w/v) |
|---|---|
| Eucheuma Carrageenan | 0.8 |
| Tobramycin | 2.0 |
| Cellulose Phosphate | q.s. to pH 7.4 (5.2) |
| Purified Water | q.s. to 100% |

To a screwcap flask was added 1 g of tobramycin and 30 ml of deionized water. After stirring and dissolution, the pH was about 10. Sufficient (typically, 2.6 g) cellulose phosphate (acid) was added to reduce the pH to 7.4. The eucheuma, 0.4 g, was added while stirring and then water added to bring the final volume to 50 ml. The capped flask was heated to 90°C and stirred for 30 minutes. Upon cooling, the pH was no lower than 7.

### Example 14

The following procedure was utilized to prepare a 50 ml batch of tobramycin formulation.

| INGREDIENT | AMOUNT (%w/v) |
|---|---|
| Eucheuma Carrageenan | 0.8 |
| Tobramycin | 2.0 |
| Carboxymethyl Sephadex® | q.s. to pH 7.4 (4.0) |
| Purified Water | q.s. to 100% |

To a screwcap flask was added 1 g of tobramycin and 30 ml of deionized water. After stirring and dissolution, the pH was about 10. Sufficient (typically, 2 g) Carboxymethyl Sephadex® (acid) was added to reduce the pH to 7.4. The eucheuma, 0.4 g, was added while stirring and then water added to bring the final volume to 50 ml. The capped flask was heated to 90°C and stirred for 30 minutes. Upon cooling, the pH was no lower than 7.

### Example 15

The following procedure was utilized to prepare a 50 ml batch of tobramycin formulation.

| INGREDIENT | AMOUNT (%w/v) |
|---|---|
| Eucheuma Carrageenan | 0.8 |
| Tobramycin | 2.0 |
| Sulfopropyl Sephadex® | q.s. to pH 7.4 (7.2) |
| Purified Water | q.s. to 100% |

To a screwcap flask was added 1 g of tobramycin and 30 ml of deionized water. After stirring and dissolution, the pH was about 10. Sufficient (typically, 3.6 g) Sulfopropyl Sephadex® (acid) was added to reduce the pH to 7.4. The eucheuma, 0.4 g, was added while stirring and then water added to bring the final volume to 50 ml. The capped flask was heated to 90°C and stirred for 30 minutes. Upon cooling, the pH was no lower than 7.

### Example 16

The following procedure was utilized to prepare a 50 ml batch of tobramycin formulation.

| INGREDIENT | AMOUNT (%w/v) |
|---|---|
| Eucheuma Carrageenan | 0.8 |
| Tobramycin | 2.0 |
| Na₂HPO₄ | 0.06 |
| KH₂PO₄ | 0.03 |
| Amberlite® IRP-64 | q.s. to pH 7.4 (3.0) |
| Purified Water | q.s. to 100% |

To a screwcap flask were added 1 g of tobramycin, 32 mg of Na₂HPO₄, 14 mg of KH₂PO₄ and 1.5 g of ball-milled Amberlite® IRP-64, followed by 30 ml of deionized water. The suspension was equilibrated for 16 hours, during which time the pH was 7. Eucheuma carrageenan, 0.4 g, was added while the suspension was stirred and then water added to bring the final volume to 50 ml. The capped flask was heated to 90°C and stirred for 30 minutes. Upon cooling, the pH was no lower than 7.

### Example 17

The following procedure was utilized to prepare a 50 ml batch of diclofenac formulation.

| INGREDIENT | AMOUNT (%w/v) |
|---|---|
| Eucheuma Carrageenan | 0.8 |
| Diclofenac | 1.0 |
| Na₂HPO₄ | 0.1 |
| Mannitol | 3.5 |
| Duolite® AP-143 | 5.0 |
| NaOH or HCl | q.s. to pH 7.4 |
| Purified Water | q.s. to 100% |

Water (30 ml), Na₂HPO₄, mannitol, and 2.5 g of basic Duolite AP-143 were added to a flask. Diclofenac, 1 %w/v, was added and the mixture stirred approximately 36 hours without adjusting pH. The pH of the mixture was then adjusted to 7.4 by addition of 1.0 N HCl, and water added to bring the volume to 45 ml. The subsequent treatment of this mixture was the same as that described in Example 1, but with addition of 0.4 g eucheuma rather than 1.0 g.

### Example 18

The following procedure was utilized to prepare a 50 ml batch of (carbonic anhydrase inhibitor) ALO4414 formulation.

| INGREDIENT | AMOUNT (%w/v) |
|---|---|
| Gelrite® | 0.6 |
| ALO4414 | 2.0 |
| Na₂HPO₄ | 0.1 |
| Mannitol | 3.5 |
| Amberlite® IRP-69 | 5.0 |
| NaOH or HCl | q.s. to pH 7.4 |
| Purified Water | q.s. to 100% |

Water, Na₂HPO₄, mannitol and 2.5 g of Amberlite® IRP-69 were added as in Example 1, and pH adjusted as described. ALO4414 (base), 2 %w/v, was added and the mixture stirred approximately 1/2 hour without adjusting pH. The pH of the mixture was then adjusted to 3 by addition of 1.0 N NaOH. The subsequent treatment of this mixture was the same as that described in Example 11.

### Example 19

The following procedure was utilized to prepare a 50 ml batch of apraclonidine formulation.

| INGREDIENT | AMOUNT (%w/w) |
|---|---|
| Eucheuma carrageenan | 1.65 |
| Apraclonidine | 0.5 |
| Amberlite® IRP-69 | 3.0 |
| Mannitol | 2.9 |
| Maleic Acid | 0.27 |
| TRIS* | q.s. to pH 6.5 (1.61) |
| Purified Water | q.s. to 100% |

| | |
|---|---|
| *Tromethamine | |

First, a concentrated solution of 2.2% eucheuma was compounded (solution A) as follows: 685 g of water was added to a 1 liter container, which was capped tightly and heated for 30 minutes in an oven set at 90°C. While stirring vigorously, 15.4 g of eucheuma was added. Stirring continued for another 30 minutes while heating at 90°C. The solution was filtered while hot through a 5µm filter (142 mm diameter) (available from MSI, Westboro, MA) under moderate pressure (30 pounds per square inch (psi)) to remove unwanted foreign material. This filtered solution was stored in a refrigerator until it was required for later compounding.

Next, a suspension was compounded containing Amberlite® IRP-69 acid, apraclonidine (base), TRIS, maleic acid and mannitol. The following were added to a 250 ml screwcap bottle: 21.8 g of deionized water, 765 mg of apraclonidine (base) and 4.5 9 of Amberlite® IRP-69. This suspension was gently agitated on an orbit shaker bath (150 rpm) for 36 hours. Next, the following ingredients were added: 4 mg of maleic acid, 2.3 g of TRIS and 4.3 g of mannitol. After all soluble ingredients were dissolved, the pH was measured to be 6.1. TRIS solution, 500µl of 1 N, was added to bring the solution pH to 6.5. Sufficient water was added to bring the total weight of this concentrated suspension to 37.6 g. This suspension constituted solution B and contained 12% Amberlite® IRP-69, 2.0% apraclonidine, 6.4% TRIS, 1.07% maleic acid, and 11.6% mannitol (w/w).

Finally, 37.5 g of Solution A (approximately 3 parts) was added to 12.5 g of solution B (approximately 1 part); this mixture was heated with stirring for 30 minutes. The pH was found to be 6.7 and the osmolality was 280 mOsm at this point. The formulation was sterilized by autoclaving the 250 ml screwcap container at 132°C for 5 minutes and was transferred to sterile ophthalmic droptainers using aseptic methods. The formulation displayed thixotropic characteristics which prevented the settling of the resin upon storage, but with agitation was demonstrated to be dropwise dispensable through a dropper tip.

### Example 20

The following procedure was utilized to prepare a 50 ml batch of ALO4414 formulation.

| INGREDIENT | AMOUNT (%w/w) |
|---|---|
| Eucheuma carrageenan | 1.5 |
| ALO4414 | 1.0 |
| Amberlite® IRP-69 | 3.0 |
| Mannitol | 3.3 |
| Boric Acid | 0.4 |
| TRIS | q.s. to pH 6.5 (1.1) |
| Purified Water | q.s. to 100% |

ALO4414 (base), 0.5 g, 1.5 g of Amberlite® IRP-69 and 30 ml of deionized water were added to a vial. This vial was agitated on an orbit shaker bath (150 rpm). After shaking for 46 hours, boric acid (0.2 g) and mannitol (1.65 g) were added. The pH was adjusted to 6.5 by adding 1 N TRIS solution dropwise. Eucheuma, 0.75 g, and enough deionized water were added to bring the solution weight to 50 g. The formulation was stirred while heating in an oil bath equilibrated at 90°C for 30 minutes. When cooled, the formulation exhibited thixotropic character, was dropwise dispensable and formed spherical gelled masses when dispensed into a model artificial tear solution.

### Example 21

The following procedure was utilized to prepare a 50 ml batch of S-betaxolol formulation.

| INGREDIENT | AMOUNT (%w/v) |
|---|---|
| Eucheuma carrageenan | 0.3 |
| S-betaxolol | 0.5 |
| Na₂HPO₄ | 0.1 |
| Amberlite® IRP-69 | 5.0 |
| Mannitol | 3.5 |
| NaOH or HCl | q.s. to pH 7.4 |
| Purified Water | q.s. to 100% |

Water, Na₂HPO₄, mannitol, and 2.5 g of Amberlite® IRP-69 were added as in Example 1, and pH adjusted as described. S-betaxolol (base), 0.5 %w/v, was added and the mixture stirred approximately 1/2 hour without adjusting pH. The pH of the mixture was then adjusted to 3 by addition of 1.0 N NaOH. The subsequent treatment of this mixture was the same as that described in Example 1, but with addition of 0.15 g eucheuma rather than 1.0 g.

### Example 22

The following procedure was utilized to prepare a 50 ml batch of apraclonidine formulation.

| INGREDIENT | AMOUNT (%w/v) |
|---|---|
| K-carrageenan | 0.5 |
| Apraclonidine | 1.0 |
| Na₂HPO₄ | 0.1 |
| Amberlite® IRP-69 | 3.0 |
| Mannitol | 3.5 |
| NaOH or HCl | q.s. to pH 7.4 |
| Purified Water | q.s. to 100% |

Water, Na₂HPO₄, mannitol and 1.5 g of Acid Amberlite® were added as in Example 1 and pH adjusted as described. Apraclonidine (base), 1 %w/v, was added and the mixture stirred approximately 1/2 hour without adjusting pH. The pH of the mixture was then adjusted to 3 by addition of 1.0 N NaOH. The subsequent treatment of this mixture was the same as that described in Example 1, but with addition of 0.25 g K-carrageenan rather than 1.0 g of eucheuma carrageenan.

### Example 23

The following procedure was utilized to prepare a 50 ml batch of ALO4414 formulation.

| INGREDIENT | AMOUNT (%w/v) |
|---|---|
| Eucheuma carrageenan | 0.3 |
| ALO4414 | 2.0 |
| Na₂HPO₄ | 0.1 |
| Amberlite® IRP-69 | 5.0 |
| Mannitol | 3.5 |
| NaOH or HCl | q.s. to pH 7.4 |
| Purified Water | q.s. to 100% |

Water, Na₂HPO₄, mannitol, and 2.5 g of Amberlite® IRP-69 were added as in Example 1 and pH adjusted as described. ALO4414 (base), 2 %w/v, was added and the mixture stirred approximately 1/2 hour without adjusting pH. The pH of the mixture was then adjusted to 3 by addition of 1.0 N NaOH. The subsequent treatment of this mixture was the same as that described in Example 21.

### Example 24

The following procedure was utilized to prepare a 50 ml batch of tobramycin formulation.

| INGREDIENT | AMOUNT (%w/v) |
|---|---|
| Furcellaran | 0.3 |
| Tobramycin | 2.0 |
| Cellulose Phosphate | q.s. to pH 7.4 (5.2) |
| Purified Water | q.s. to 100% |

This example was prepared in a manner identical with that of Example 4, with the exception that 0.15 g of furcellaran was substituted for 1 g of eucheuma.

### Example 25

The following procedure was utilized to prepare a 50 ml batch pilocarpine formulation.

| INGREDIENT | AMOUNT (%w/v) |
|---|---|
| Eucheuma carrageenan | 0.3 |
| Pilocarpine | 1.0 |
| Na₂HPO₄ | 0.1 |
| Amberlite® IRP-69 | 5.0 |
| Mannitol | 3.5 |
| NaOH or HCl | q.s. to pH 7.4 |
| Purified Water | q.s. to 100% |

Water, Na₂HPO₄, mannitol, and 2.5 g of Amberlite® IRP-69 were added as in Example 1, and pH adjusted as described. Pilocarpine (base), 1 %w/v, was added and the mixture stirred approximately 1/2 hour without adjusting pH. The pH of the mixture was then adjusted to 3 by addition of 1.0 N NaOH. The subsequent treatment of this mixture was the same as that described in Example 21.

### Example 26

The following procedure was utilized to prepare a 50 ml batch pilocarpine formulation.

| INGREDIENT | AMOUNT (%w/v) |
|---|---|
| Gelrite® | 0.3 |
| Pilocarpine | 1.0 |
| Na₂HPO₄ | 0.1 |
| Amberlite® IRP-69 | 5.0 |
| Mannitol | 3.5 |
| NaOH or HCl | q.s. to pH 7.4 |
| Purified Water | q.s. to 100% |

Water (30 ml), Na₂HPO₄, mannitol, 2.5 g of Amberlite® IRP-64 and pilocarpine (base), 1 %w/v, were added and the mixture stirred approximately 12 hours without adjusting pH. The pH of the mixture was then adjusted to 7.4 by addition of 1.0 N NaOH and water added to bring the volume to 45 ml. The subsequent treatment of this mixture was the same as that described in Example 1, except that 0.15 g of Gelrite® was used in place of 1 g of eucheuma.

### Example 27

The following procedure was utilized to prepare a 50 ml batch of S-betaxolol formulation.

| INGREDIENT | AMOUNT (%w/v) |
|---|---|
| K-carrageenan | 0.5 |
| S-betaxolol HCl | 0.56 |
| Na₂HPO₄ | 0.1 |
| Gelwhite® | 3.0 |
| Mannitol | 3.5 |
| NaOH or HCl | q.s. to pH 7.4 |
| Purified Water | q.s. to 100% |

This example was compounded in a manner identical with that of Example 9, with the exceptions that 0.1% Na₂HPO₄ was added, Gelwhite® was used in place of bentonite, and 0.25 g of K-carrageenan was used in place of 1.0 g of eucheuma.

### Example 28

The following procedure was utilized to prepare a 50 ml batch of diclofenac formulation.

| INGREDIENT | AMOUNT (%w/v) |
|---|---|
| Furcellaran | 0.3 |
| Diclofenac | 1.0 |
| Na₂HPO₄ | 0.1 |
| Duolite® AP-143 | 5.0 |
| Mannitol | 3.5 |
| NaOH or HCl | q.s. to pH 7.4 |
| Purified Water | q.s. to 100% |

Water (30 ml), Na₂HPO₄, mannitol and 2.5 g of Duolite® AP-143 base were to a flask. Diclofenac (acid), 1 %w/v, was added and the mixture stirred approximately 36 hours without adjusting pH. The pH of the mixture was then adjusted to 7.4 by addition of 1.0 N HCl and water added to bring the volume to 45 ml. The subsequent treatment of this mixture was the same as that described in Example 24.

### Example 29

The following five S-betaxolol formulations were prepared to demonstrate the synergy derived from coupling gelling polysaccharides with drug carrier substrates to increase bioavailability by sustaining the residence in the precorneal area. The examples are meant to be illustrative of the phenomena and are not intended to be limited to betaxolol; the findings can be applied broadly.

| | FORMULATION (%w/v) | | | | |
|---|---|---|---|---|---|
| INGREDIENT | A | B | C | D | E |
| Eucheuma Carrageenan | 1.2 | 1.2 | 1.2 | --- | --- |
| S-betaxolol (base) | 1.0 | 1.0 | --- | 1.0 | --- |
| S-betaxolol HCl | --- | --- | 1.12 | --- | 1.12 |
| Na₂HPO₄ | 0.1 | 0.1 | 0.1 | 0.1 | 0.1 |
| Amberlite® IRP-69 | 10.0 | 1.0 | --- | 1.1 | --- |
| NaOH or HCl | q.s. to pH 7.4 | | | | |
| Purified Water | q.s. to 100% | | | | |

The compounding procedures described below were utilized to prepare 50 ml batches of Formulations A-E.

### Formulation A

Water, buffer, and 5.0 g of Amberlite® IRP-69 were added as in Example 1, and pH adjusted as described. Betaxolol (base), 1 %w/v, was added and the mixture stirred approximately 1/2 hour without adjusting pH. The pH of the mixture was then adjusted to 3 by addition of 1.0 N NaOH. The subsequent treatment of this mixture was the same as that described in Example 1, except that only 0.6 g of eucheuma was added.

### Formulation B

The same procedure was used to make this formulation as Formulation A, except that 0.5 g of resin was used and the pH was adjusted only after equilibration.

### Formulation C

Water, Na₂HPO₄ and S-betaxolol HCl, 1.12 %w/v, were added, the mixture stirred and the pH was adjusted to 7.4. Eucheuma, 0.6 g, was then added. Sufficient water was added to bring the final volume to 50 ml and the solution was heated to about 90°C with stirring for 30 minutes. This solution was injected into a mold (see US 4,871,094) while hot and sealed with polypropylene-backed foil. Upon cooling, a cylindrical insert (having approximate dimensions of 1 mm diameter x 8 mm length) was formed in the mold. Note that in the absence of resin, the drug interaction with the eucheuma was sufficient to gel the formulation without any added salt.

### Formulation D

Water, Na₂HPO₄ and 0.55 g of Amberlite® IRP-69 were added as in Example 1, and pH adjusted as described. S-betaxolol (base), 1 %w/v, was added and the mixture stirred approximately 12 hours without adjusting pH. The pH of the mixture was then adjusted to 7.4 with 10 N NAOH.

### Formulation E

Water, Na₂HPO₄ and 1.12% of S-betaxolol HCl were mixed and stirred until drug was dissolved, then the pH was adjusted to 7.4.

**In vitro** **Time Release Measurements.** There is used herein in vitro methodology which closely simulates the hydrodynamics of the precorneal fluid occurring in vivo, called a Controlled Release Analytical System (CRAS). (Stevens et al., Analytical Chemistry, 64:715 (1992).) The CRAS reproduces the small volume (approximately 10-30 µl) and slow exchange rate (approximately 1 µl/min) of the tear reservoir in the human cul-de-sac. These experimental features are vitally important for evaluating time release profiles of formulations which sustain drug release, such as gels and suspensions claimed herein. For one of the systems, the gelable drop, preliminary in vivo release data corroborating the time release rate measured in vitro was included.

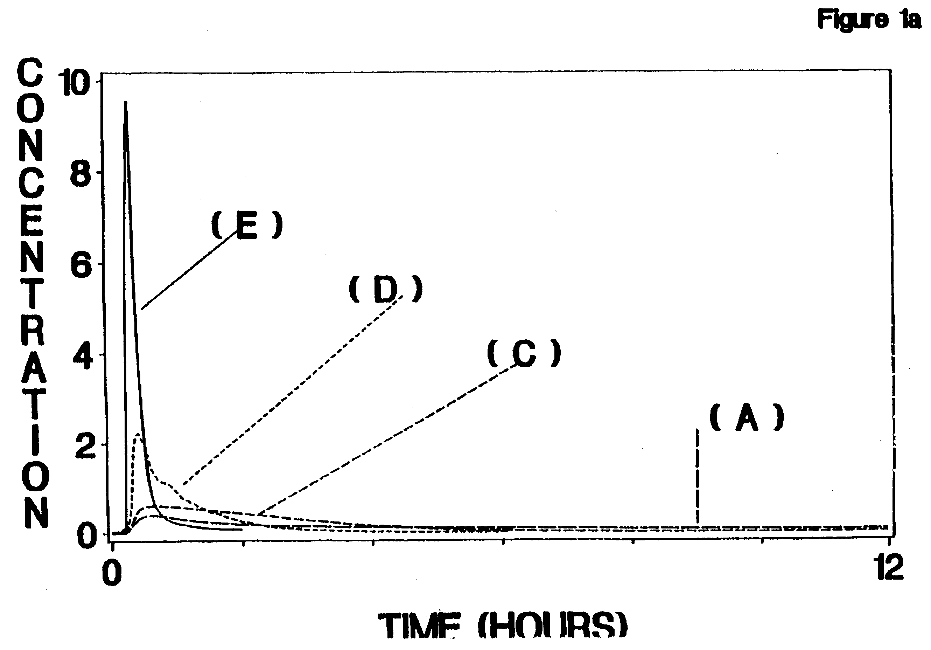

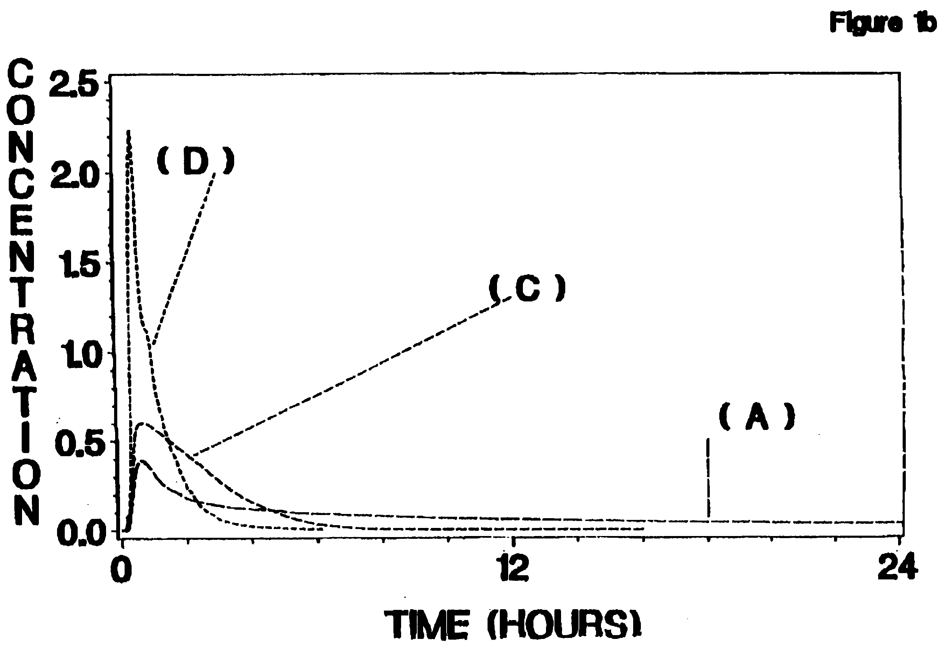

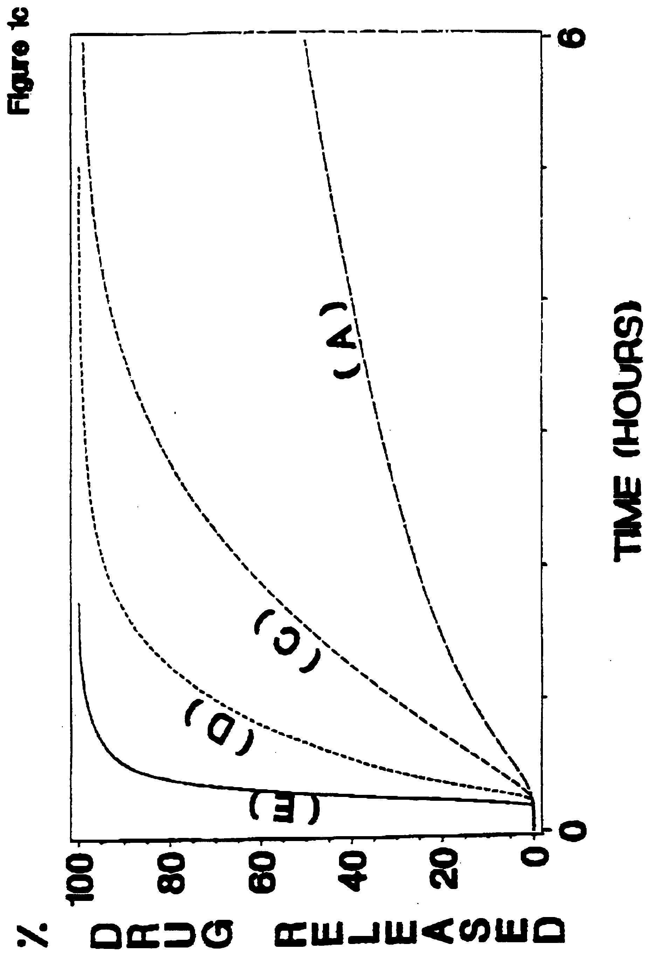

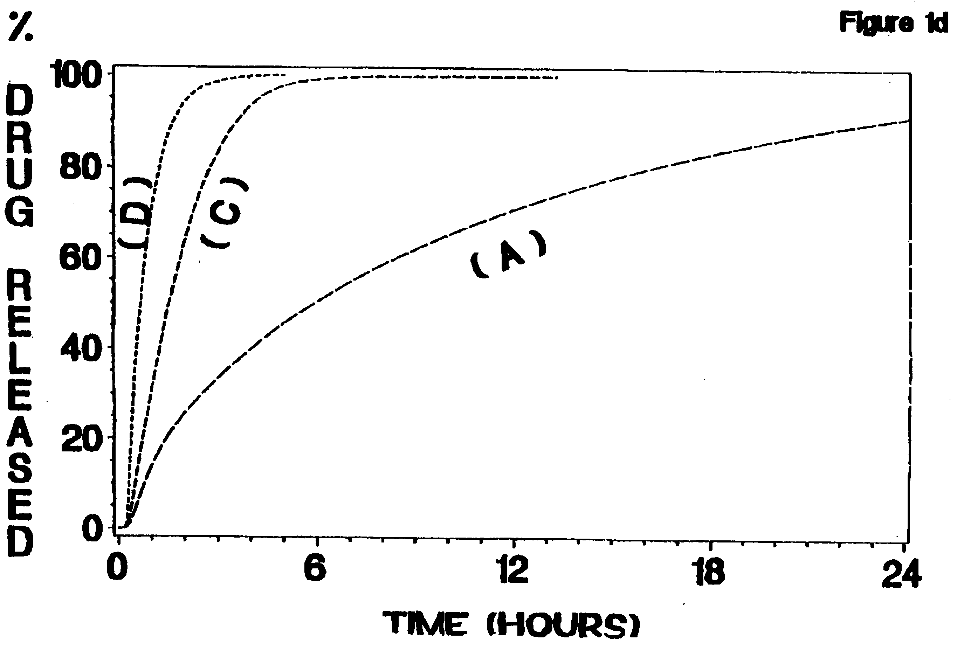

Figure 1a accompanying the application, shows the concentration-time profiles for four of the widely different types of ophthalmic formulations used. Figure 1a emphasizes the vast difference in release rates between the solution (Formulation E) and the other sustained release compositions, whereas Figure 1b shows a more detailed comparison of the sustained release formulations.

The integrals of the concentration-time profiles are presented in Figures 1c and 1d: (1c showing the integral for early times and 1d showing later times), from which the drug residence half-life is determinable. The integrated concentration-time profile half-lives of the solution (Formulation E), the suspension (Formulation D), the resinless gel insert (Formulation C), and the two gelable drop solutions, one with more (Formulation A) and one with less (Formulation B) resin (see Table 1), were 7 minutes, 27 minutes, 1-1/3 hours, 6 hours, and 2.5 hours, respectively. Concomitant with this sustained delivery is a reduction in the peak in-use concentration which should decrease unwanted side effects such as discomfort while improving the therapeutic ratio.

These formulations and release parameters are summarized in the following Table 1. Data for two preparations of apraclonidine, Formulations F and G, are also cited to indicate the technology can be applied broadly.

**Table 1**

| SUMMARY OF FORMULATIONS IN CRAS ANALYSIS | | | | | | | |
|---|---|---|---|---|---|---|---|
| | FORMULATIONS (%w/v) | | | | | | |
| INGREDIENT | A | B | C | D | E | F | G |
| Eucheuma Carrageenan | 1.2 | 1.2 | 1.2 | --- | --- | 1.5 | --- |
| Na₂HPO₄ | 0.1 | 0.1 | 0.1 | 0.1 | 0.1 | --- | --- |
| Amberlite® IRP-69 | 10.0 | 1 | --- | 1.1 | --- | 3.0 | 3.0 |
| S-betaxolol base | 1.0 | 1.0 | --- | 1.0 | --- | --- | --- |
| S-betaxolol HCl | --- | --- | 1.12 | --- | 1.12 | --- | --- |
| Apraclonidine | --- | --- | --- | --- | --- | 0.5 | 0.5 |
| NaOH or HCl | q.s. to pH 7.4 | | | | | --- | --- |
| TRIS | --- | --- | --- | --- | --- | 1.2* | 1.2* |
| Maleic Acid | --- | --- | --- | --- | --- | 0.12 | 0.12 |
| Water | q.s. to 100% | | | | | | |
| | | | | | | | |
| t_{1/2} (hours) | 6 | 2.5 | 1.3 | 0.45 | 0.12 | 6 | 2 |

| | | | | | | | |
|---|---|---|---|---|---|---|---|
| *q.s. to pH 6.5 | | | | | | | |

### In vivo Time Release Measurements

In an ocular bioavailability experiment, the time release of radiolabelled S-betaxolol from two gelable drop formulations, A and B, were measured. The two formulations were chosen to provide a contrast in drug release rates, with Formulation B, having only a tenth the resin of Formulation A, having faster release. Samples were inserted in rabbit cul-de-sacs and removed after two and six hours using cotton swabs. Portions of the retrieved samples were digested in 2 ml of 0.2 M trifluoroacetic acid for 24 hours at 90°C and then neutralized with 435 µl 1 N NaOH. An HPLC method was used to measure the amount of galactose retrieved from this digestion as a measure of the mass of polysaccharide present. S-betaxolol content was measured by scintillation counting. In this way, the amount of S-betaxolol measured in each sample could be normalized by the amount of gel retrieved to obtain an approximate measure of the time dependence of the fraction of drug released after instillation.

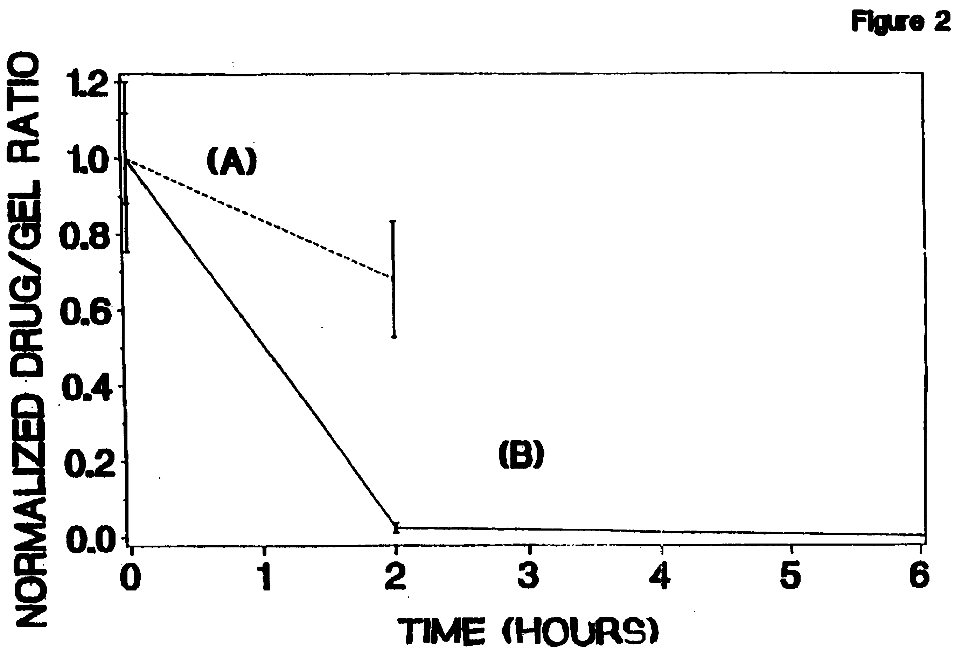

The in vivo release data are shown in Figure 2 for both gelable drop formulations. After two hours residence time, 31 ± 15% of the drug has been released from gelable drop, Example A, whereas > 95% of the drug has been released from the second gelable drop, Formulation B, with a tenfold decrease in the level of resin. From the cumulative time release profile of Figure 1c for Formulation A, about 29% of the drug had been released in vitro after two hours. A similar profile has been measured for a gelable drop formulation quite similar to Formulation B, which released ≧ 50% of its drug after two hours in the CRAS. Good agreement was found between in vivo and in vitro release profiles.

### Summary

As described above, the use of DCS in sustaining drug release increases bioavailability while diminishing unwanted side effects. On the other hand, a gel vehicle used alone can increase bioavailability by extending the residence time in the precorneal area. The present invention combines these two approaches so that duration of drug release could be sustained further, as compared to either approach alone. The approach, illustrated for S-betaxolol and for apraclonidine, but applicable to a variety of drugs which can be bound by ion-exchange resin or confined in other drug carriers for subsequent release, provides a general way in which value can be added to existing drug systems.

Another feature, a biological synergy, is the observed extended residence of drug carrier substrates when they are incorporated into a gel rather than presented as a suspension. The synergy is not only apparent in the time release profiles and extended residence, but also in another important feature - vehicle aesthetics. If no DCS is used for a cationic drug such as S-betaxolol, the free drug solution is sufficient to set the gel even before administration. This creates a very resilient gel which has excellent retention properties (for comfortable drugs), yet which must be treated as an ocular insert rather than a dropable solution. By adding sufficient DCS, the free drug concentration can be lowered to the point where the mixture is flowable and can be dispensed as a drop. Thus, the addition of ion-exchange resin enlarges the class of materials which can be delivered by the method of the present invention. Since one would normally expect an increase in viscosity rather than a decrease upon addition of a dispersed solid phase, this phenomenon is both unique and unexpected.

Thus, the independent use of either DCS or gelling polysaccharide provide benefits in sustaining drug release while reducing the peak in- use concentration associated with ophthalmic dosage forms. The present invention combines these two technologies and provides a particularly useful strategy for extending the release and improving aesthetics (and thereby compliance).

## Claims (Claims for the following Contracting State(s): AT, BE, CH, LI, DE, DK, FR, GB, IT, LU, MC, NL, PT, SE)

1. A topical, ophthalmic, sustained release composition comprising a mixture of a drug, a gelling polysaccharide and a finely-divided drug carrier substrate, wherein the concentration of said gelling polysaccharide is such that the composition is administrable as a drop and gels upon instillation.

2. The composition of claim 1, wherein the finely-divided drug carrier substrate is an ion exchange resin.

3. The Composition of claim 1, wherein the gelling polysaccharide concentration is between about 0.1% to about 3.0% by weight/volume and the concentration of the finely-divided drug carrier substrate is between about 0.05% to about 10.0% by weight/volume.

4. The composition of claim 1, wherein the gelling polysaccharide is selected from the group consisting of: xanthan gum, locust bean gum, gellan gum and carrageenan.

5. The composition of claim 4, wherein the gelling polysaccharide is a carrageenan having not more than 1.0 sulfate moiety per disaccharide repeating unit.

6. The composition of claim 4, wherein said carrageenan is eucheuma carrageenan or furcellaran.

7. The composition of claim 1, wherein the pre-dosed viscosity is in the range of about 1 to about 5000 mPa·s (cps) and the post-dosed viscosity is greater than about 50 mPa·s (cps).

8. The composition of claim 1 wherein the pre-dosed viscosity is in the range of about 1 to about 500 mPa·s (cps) and the post-dosed gel has a modulus of elasticity in the range of 1 X 10⁴ to about 5 X 10⁵ dPa (dynes/cm²).

9. The composition of claim 1, wherein the pre-dosed modulus of elasticity is in the range of about 1 X 10⁴ to about 3 X 10⁵ dPa (dynes/cm²) and the post-dosed modulus of elasticity is in the range of about 1 X 10⁴ to about 2 X 10⁶ dPa (dynes/cm²).

10. The composition of claim 1, wherein the composition is thixotropic having a pre-dosed modulus of elasticity in the range of about 1 X 10⁴ to about 2 X 10⁵ dPa (dynes/cm²) and when shaken, a pre-dosed viscosity in the range of about 1 to about 5000 mPa·s (cps) and having a post-dosed modulus of elasticity in the range of about 1 X 10⁴ to about 2 X 10⁶ dPa (dynes/cm²).

11. The composition of claim 1, wherein the drug is selected from the group consisting of: glaucoma agents, anti-hypertensives, non-steroidal and steroidal anti-inflammatory agents, anti-bacterials and anti-infectives, anti-fungals, anti-virals, anti-cataract agents, anti-oxidants, anti-allergics, growth factors and prodrugs thereof.

12. The composition of claim 11, wherein the glaucoma agent is selected from the group consisting of: β-blockers, α-agonists, carbonic anhydrase inhibitors, dopamine agonists and antagonists, miotic cholinergics, prostaglandins, ACE inhibitors, steroids and calcium channel blockers.

13. The composition of claim 11, wherein the drug is selected from the group consisting of the racemic and enantiomeric forms of: betaxolol, timolol, carteolol, apraclonidine, (+)-4-ethylamino-2,3-dihydro-4-H-2-methylthieno[3,2-e]-1,2-thiazine-6-sulfonamide-1,1-dioxide, pilocarpine, carbachol, prostaglandins, ACE inhibitors, glucocorticoids, angiostatic steroids, calcium channel blockers, diclofenac, ketorolac, fluoromethalone acetate, prednisolone acetate, tetrahydrocortisol, tobramycin, quinolones, beta-lactams, natamycin, acyclovir and ganciclovir.

14. Use of a topical, ophthalmic, sustained release composition for delivering a drug to eyes, comprising a mixture of a drug, a gelling polysaccharide and a finely-divided drug carrier substrate, wherein the concentration of said gelling polysaccharide is such that the composition is administrable as a drop and gels upon instillation for the manufacture of a medicament for topically delivering a drug to eyes.

15. The Use of claim 14, wherein the gelling polysaccharide concentration is between about 0.1 % to about 3.0 % by weight/volume and the finely-divided drug carrier substrate concentration is between about 0.05 % to about 10.0 % by weight.

16. The use of claim 15, wherein the gelling polysaccharide is selected from the group consisting of: xanthan gum, locust bean gum, gellan gum and carrageenan and the finely divided drug carrier substrate is an ion exchange resin.

17. The use of claim 16, wherein said carrageenan is euchema carrageenan or furcellaran, and the drug is para-amino clonidine or a carbonic anhydrase inhibitor.

18. The use of claim 17, wherein the carbonic anhydrase inhibitor is (+)-4-ethylamino-2,2-dihydro-4-H-2-methylthieno[3,2-e]-1,2-thiazine-6-sulfonamide-1,1-dioxide or a pharmaceutically acceptable salt thereof.

19. The composition of claim 1, wherein the finely-divided drug carrier substance is amberlite resin and the gelling polysaccharide is eucheuma.

20. The use of claim 14, wherein the finely-divided drug carrier substance is amberlite resin and the gelling polysaccharide is eucheuma.

21. A non-drug containing topical ophthalmic composition comprising a gelling polysaccharide and a finely-divided drug carrier substrate, wherein the concentration of said gelling polysaccharide is such that the composition is administrable as a drop and gels upon instillation.

22. The composition of claim 21, wherein the finely-divided drug carrier substrate is an ion exchange resin.

23. The composition of claim 21, wherein the gelling polysaccharide concentration is between about 0.1% to about 3.0% by weight/volume and the concentration of the finely-divided drug carrier substrate is between about 0.05% to about 10.0% by weight/volume.

24. The composition of claim 21, wherein the gelling polysaccharide is selected from the group consisting of: xanthan gum, locust bean gum, gellan gum and carrageenan.

25. The composition of claim 24, wherein the gelling polysaccharide is a carrageenan having not more than 1.0 sulfate moiety per disaccharide repeating unit.

26. The composition of claim 24, wherein said carrageenan is eucheuma carrageenan or furcellaran.

27. The composition of claim 21, wherein the pre-dosed viscosity is in the range of about 1 to about 5000 mPa·s (cps) and the post-dosed viscosity is greater than about 50 mPa·s (cps).

28. The composition of claim 21, wherein the pre-dosed viscosity is in the range of about 1 to about 500 mPa·s (cps) and the post-dosed gel has a modulus of elasticity in the range of 1 X 10⁴ to about 5 X 10⁵ dPa (dynes/cm²).

29. The composition of claim 21, wherein the pre-dosed modulus of elasticity is in the range of about 1 X 10⁴ to about 3 X 10⁵ dPa (dynes/cm²) and the post-dosed modulus of elasticity is in the range of about 1 X 10⁴ to about 2 X 10⁶ dPa (dynes/cm²).

30. The composition of claim 21, wherein the composition is thixotropic having a pre-dosed modulus of elasticity in the range of about 1 X 10⁴ to about 2 X 10⁵ dPa (dynes/cm²) and, when shaken, a pre-dosed viscosity in the range of about 1 to about 5000 mPa·s (cps) and having a post-dosed modulus of elasticity in the range of about 1 X 10⁴ to about 2 X 10⁶ dPa (dynes/cm²).

31. Use of a non-drug containing ophthalmic composition, comprising a gelling polysaccharide and a finely-divided drug carrier substrate, wherein the concentration of said gelling polysaccharide is such that the composition is administrable as a drop and gels upon instillation according to anyone of claims 21 - 30 for the manufacture of a medicament for lubricating the eye or supplementing tears to the eye.

## Claims (Claims for the following Contracting State(s): ES, GR)

1. A method for preparing a topical, ophthalmic, sustained release composition comprising mixing of a drug, a gelling polysaccharide and a finely-divided drug carrier substrate, wherein the concentration of said gelling polysaccharide is such that the composition is administrable as a drop and gels upon instillation to form a topical, ophthalmic, sustained release composition.

2. The method of claim 1, wherein the finely-divided drug carrier substrate is an ion exchange resin.

3. The method of claim 1, wherein the gelling polysaccharide concentration is between about 0.1% to about 3.0% by weight/volume and the concentration of the finely-divided drug carrier substrate is between about 0.05% to about 10.0% by weight/volume.

4. The method of claim 1, wherein the gelling polysaccharide is selected from the group consisting of: xanthan gum, locust bean gum, gellan gum and carrageenan.

5. The method of claim 4, wherein the gelling polysaccharide is a carrageenan having not more than 1.0 sulfate moiety per disaccharide repeating unit.

6. The method of claim 4, wherein said carrageenan is eucheuma carrageenan or furcellaran.

7. The method of claim 1, wherein the pre-dosed viscosity is in the range of about 1 to about 5000 mPa·s (cps) and the post-dosed viscosity is greater than about 50 mPa·s (cps).

8. The method of claim 1 wherein the pre-dosed viscosity is in the range of about 1 to about 500 mPa·s (cps) and the post-dosed gel has a modulus of elasticity in the range of 1 X 10⁴ to about 5 X 10⁵ dPa (dynes/cm²).

9. The method of claim 1, wherein the pre-dosed modulus of elasticity is in the range of about 1 X 10⁴ to about 3 X 10⁵ dPa (dynes/cm²) and the post-dosed modulus of elasticity is in the range of about 1 X 10⁴ to about 2 X 10⁶ dPa (dynes/cm²).

10. The method of claim 1, wherein the composition is thixotropic having a pre-dosed modulus of elasticity in the range of about 1 X 10⁴ to about 2 X 10⁵ dPa (dynes/cm²) and, when shaken, a pre-dosed viscosity in the range of about 1 to about 5000 mPa·s (cps) and having a post-dosed modulus of elasticity in the range of about 1 X 10⁴ to about 2 X 10⁶ dPa (dynes/cm²).

11. The method of claim 1, wherein the drug is selected from the group consisting of: glaucoma agents, anti-hypertensives, non-steroidal and steroidal anti-inflammatory agents, anti-bacterials and anti-infectives, anti-fungals, anti-virals, anti-cataract agents, anti-oxidants, anti-allergics, growth factors and prodrugs thereof.

12. The method of claim 11, wherein the glaucoma agent is selected from the group consisting of: β-blockers, α-agonists, carbonic anhydrase inhibitors, dopamine agonists and antagonists, miotic cholinergics, prostaglandins, ACE inhibitors, steroids and calcium channel blockers.

13. The method of claim 11, wherein the drug is selected from the group consisting of the racemic and enantiomeric forms of: betaxolol, timolol, carteolol, apraclonidine, (+)-4-ethylamino-2,3-dihydro-4-H-2-methylthieno[3,2-e]-1,2-thiazine-6-sulfonamide-1,1-dioxide, pilocarpine, carbachol, prostaglandins, ACE inhibitors, glucocorticoids, angiostatic steroids, calcium channel blockers, diclofenac, ketorolac, fluoromethalone acetate, prednisolone acetate, tetrahydrocortisol, tobramycin, quinolones, beta-lactams, natamycin, acyclovir and ganciclovir.

14. Use of a topical, ophthalmic, sustained release composition for delivering a drug to eyes, comprising a mixture of a drug, a gelling polysaccharide and a finely-divided drug carrier substrate, wherein the concentration of said gelling polysaccharide is such that the composition is administrable as a drop and gels upon instillation for the manufacture of a medicament for topically delivering a drug to eyes.

15. The Use of claim 14, wherein the gelling polysaccharide concentration is between about 0.1 % to about 3.0 % by weight/volume and the finely-divided drug carrier substrate concentration is between about 0.05 % to about 10.0 % by weight.

16. The use of claim 15, wherein the gelling polysaccharide is selected from the group consisting of: xanthan gum, locust bean gum, gellan gum and carrageenan and the finely divided drug carrier substrate is an ion exchange resin.

17. The use of claim 16, wherein said carrageenan is euchema carrageenan or furcellaran, and the drug is para-amino clonidine or a carbonic anhydrase inhibitor.

18. The use of claim 17, wherein the carbonic anhydrase inhibitor is (+)-4-ethylamino-2,2-dihydro-4-H-2-methylthieno[3,2-e]-1,2-thiazine-6-sulfonamide-1,1-dioxide or a pharmaceutically acceptable salt thereof.

19. The method of claim 1, wherein the finely-divided drug carrier substance is amberlite resin and the gelling polysaccharide is eucheuma.

20. The use of claim 14, wherein the finely-divided drug carrier substance is amberlite resin and the gelling polysaccharide is eucheuma. .

21. A method for preparing a non-drug containing topical ophthalmic composition comprising mixing of a gelling polysaccharide and a finely-divided drug carrier substrate, wherein the concentration of said gelling polysaccharide is such that the composition is administrable as a drop and gels upon instillation to form a non-drug containing topical ophthalmic composition.

22. The method of claim 21, wherein the finely-divided drug carrier substrate is an ion exchange resin.

23. The method of claim 21, wherein the gelling polysaccharide concentration is between about 0.1% to about 3.0% by weight/volume and the concentration of the finely-divided drug carrier substrate is between about 0.05% to about 10.0% by weight/volume.

24. The method of claim 21, wherein the gelling polysaccharide is selected from the group consisting of: xanthan gum, locust bean gum, gellan gum and carrageenan.

25. The method of claim 24, wherein the gelling polysaccharide is a carrageenan having not more than 1.0 sulfate moiety per disaccharide repeating unit.

26. The method of claim 24, wherein said carrageenan is eucheuma carrageenan or furcellaran.

27. The method of claim 21, wherein the pre-dosed viscosity is in the range of about 1 to about 5000 mPa·s (cps) and the post-dosed viscosity is greater than about 50 mPa·s (cps).

28. The method of claim 21, wherein the pre-dosed viscosity is in the range of about 1 to about 500 mPa·s (cps) and the post-dosed gel has a modulus of elasticity in the range of 1 X 10⁴ to about 5 X 10⁵ dPa (dynes/cm²).

29. The method of claim 21, wherein the pre-dosed modulus of elasticity is in the range of about 1 X 10⁴ to about 3 X 10⁵ dPa (dynes/cm²) and the post-dosed modulus of elasticity is in the range of about 1 X 10⁴ to about 2 X 10⁶ dPa (dynes/cm²).

30. The method of claim 21, wherein the composition is thixotropic having a pre-dosed modulus of elasticity in the range of about 1 X 10⁴ to about 2 X 10⁵ dPa (dynes/cm²) and, when shaken, a pre-dosed viscosity in the range of about 1 to about 5000 mPa·s (cps) and having a post-dosed modulus of elasticity in the range of about 1 X 10⁴ to about 2 X 10⁶ dPa (dynes/cm²).

31. Use of a non-drug containing ophthalmic composition, comprising a gelling polysaccharide and a finely-divided drug carrier substrate, wherein the concentration of said gelling polysaccharide is such that the composition is administrable as a drop and gels upon instillation according to anyone of claims 21 - 30 for the manufacture of a medicament for lubricating the eye or supplementing tears to the eye.

## Patentansprüche (Patentansprüche für folgende(n) Vertragsstaat(en): AT, BE, CH, LI, DE, DK, FR, GB, IT, LU, MC, NL, PT, SE)

1. Topische ophthalmische Zusammensetzung mit verzögerter Freisetzung, umfassend eine Mischung aus einem Arzneimittel, einem gelbildenden Polysaccharid und einem fein zerteilten Arzneimittelträgersubstrat, wobei die Konzentration des gelbildenden Polysaccharids derart ist, daß die Zusammensetzung als Tropfen verabreichbar ist und beim Eintröpfeln geliert.

2. Zusammensetzung nach Anspruch 1,
wobei das fein zerteilte Arzneimittelträgersubstrat ein Ionenaustauschharz ist.

3. Zusammensetzung nach Anspruch 1,
wobei die Konzentration des gelbildenden Polysaccharids zwischen etwa 0,1 % bis etwa 3,0 % w/v und die Konzentration des fein zerteilten Arzneimittelträgersubstrats zwischen etwa 0,05 % bis etwa 10,0 % w/v liegt.

4. Zusammensetzung nach Anspruch 1,
wobei das gelbildende Polysaccharid aus der Gruppe, bestehend aus Xanthangummi, Johannisbrotgummi, Gellangummi und Carrageen ausgewählt ist.

5. Zusammensetzung nach Anspruch 4,
wobei das gelbildende Polysaccharid ein Carrageen mit nicht mehr als 1,0 Sulfatresten pro Disaccharidrepetiereinheit ist.

6. Zusammensetzung nach Anspruch 4,
wobei das Carrageen Eucheuma-Carrageen oder Furcellaran ist.

7. Zusammensetzung nach Anspruch 1,
wobei die Viskosität vor der Verabreichung im Bereich von etwa 1 bis etwa 5000 mPa.s (cps) und die Viskosität nach der Verabreichung über etwa 50 mPa.s (cps) liegt.

8. Zusammensetzung nach Anspruch 1,
wobei die Viskosität vor der Verabreichung im Bereich von etwa 1 bis etwa 500 mPa.s (cps) liegt und das Gel nach der Verabreichung ein Elastizitätsmodul im Bereich von 1 x 10⁴ bis etwa 5 x 10⁵ dPa (DYN/cm²) aufweist.

9. Zusammensetzung nach Anspruch 1,
wobei das Elastizitätsmodul vor der Verabreichung im Bereich von etwa 1 x 10⁴ bis etwa 3 x 10⁵ dPa (DYN/cm²) und das Elastizitätsmodul nach der Verabreichung im Bereich von etwa 1 x 10⁴ bis etwa 2 x 10⁶ dPa (DYN/cm²) liegt.

10. Zusammensetzung nach Anspruch 1,
wobei die Zusammensetzung thixotrop ist und ein Elastizitätsmodul im Bereich von etwa 1 x 10⁴ bis etwa 2 x 10⁵ dPa (DYN/cm²) und, nach dem Schütteln, eine Viskosität vor der Verabreichung im Bereich von etwa 1 bis etwa 5000 mPa.s (cps) und ein Elastizitätsmodul nach der Verabreichung im Bereich von etwa 1 x 10⁴ bis etwa 2 x 10⁶ dPa (DYN/cm₂) aufweist.

11. Zusammensetzung nach Anspruch 1,
wobei das Arnzeimittel aus der Gruppe, bestehend aus Glaucomamitteln, Antihypertensiva, nicht steroidalen und steroidalen Entzündungshemmern, antibakteriellen Mitteln und antiinfektiven Mitteln, antifungativen Mitteln, antiviralen Mitteln, Antikataraktmitteln, Antioxidantien, Antiallergika, Wachstumsfaktoren und deren Vorläufern ausgewählt ist.

12. Zusammensetzung nach Anspruch 11,
wobei das Glaucomamittel aus der Gruppe, bestehend aus β-Blockern, α-Agonisten, Carboanhydraseinhibitoren, Dopaminagonisten und -antagonisten, miotischen Cholinergika, Prostaglandinen, ACE-Inhibitoren, Steroiden und Calcium-Kanalblockern ausgewählt ist.

13. Zusammensetzung nach Anspruch 11,
wobei das Arzneimittel aus der Gruppe, bestehend aus den racemischen und enantiomeren Formen der nachfolgenden Verbindungen ausgewählt ist: Betaxolol, Timolol, Carteolol, Apraclonidin, (+)-4-Ethylamino-2,3-dihydro-4-H-2-methylthieno[3,2-e]-1,2-thiazin-6-sulfonamid-1,1-dioxid, Pilocarpin, Carbachol, Prostaglandinen, ACE-Inhibitoren, Glucocorticoiden, angiostatischen Steroiden, Calcium-Kanalblockern, Diclofenac, Ketorolac, Fluormethalonacetat, Prednisolonacetat, Tetrahydrocortisol, Tobramycin, Chinolonen, Beta-Lactamen, Natamycin, Acyclovir und Ganciclovir.

14. Verwendung einer topischen ophthalmischen Zusammensetzung mit verzögerter Freisetzung zur Zufuhr eines Arzneimittels zu den Augen, umfassend eine Mischung aus einem Arzneimittel, einem gelbildenden Polysaccharid und einem fein zerteilten Arzneimittelträgersubstrat, wobei die Konzentration des gelbildenden Polysaccharids derart ist, daß die Zusammensetzung als Tropfen verabreichbar ist und beim Eintröpfeln geliert, zur Herstellung eines Arzneimittels zur topischen Zufuhr eines Arzneimittels zu den Augen.

15. Verwendung nach Anspruch 14,
wobei die Konzentration des gelbildenden Polysaccharids zwischen etwa 0,1 % bis etwa 3,0 % w/v und die Konzentration des fein zerteilten Arzneimittelträgersubstrats zwischen etwa 0,05 % bis etwa 10,0 % w/v liegt.

16. Verwendung nach Anspruch 15,
wobei das gelbildende Polysaccharid aus der Gruppe, bestehend aus Xanthangummi, Johannisbrotgummi, Gellangummi und Carrageen ausgewählt ist, und das fein zerteilte Arzneimittelträgersubstrat ein Ionenaustauschharz ist.

17. Verwendung nach Anspruch 16,
wobei das Carrageen Eucheuma-Carrageen oder Furcellaran ist und der Wirkstoff para-Aminoclonidin oder ein Carboanhydraseinhibitor ist.

18. Verwendung nach Anspruch 17,
wobei der Carboanhydraseinhibitor (+)-4-Ethylamino-2,2-dihydro-4-H-2-methylthieno[3,2-e]-1,2-thiazin-6-sulfonamid-1,1-dioxid oder ein pharmazeutisch verträgliches Salz dieser Verbindung ist.

19. Zusammensetzung nach Anspruch 1,
wobei die fein zerteilte Arzneimittelträgersubstanz Amberlit-Harz ist und das gelbildende Polysaccharid Eucheuma ist.

20. Verwendung nach Anspruch 14,
wobei die fein zerteilte Arzneimittelträgersubstanz Amberlit-Harz ist und das gelbildende Polysaccharid Eucheuma ist.

21. Topische ophthalmische Zusammensetzung ohne Arzneimittel, umfassend ein gelbildendes Polysaccharid und ein fein zerteiltes Arzneimittelträgersubstrat, wobei die Konzentration des gelbildenden Polysaccharids derart ist, daß die Zusammensetzung als Tropfen verabreichbar ist und beim Einträufeln geliert.

22. Zusammensetzung nach Anspruch 21,
wobei das fein zerteilte Arzneimittelträgersubstrat ein Ionenaustauschharz ist.

23. Zusammensetzung nach Anspruch 21,
wobei die Konzentration des gelbildenden Polysaccharids zwischen etwa 0,1 % bis etwa 3,0 % w/v und die Konzentration des fein zerteilten Arzneimittelträgersubstrats zwischen etwa 0,05 % bis etwa 10,0 % w/v liegt.

24. Zusammensetzung nach Anspruch 21,
wobei das gelbildende Polysaccharid aus der Gruppe, bestehend aus Xanthangummi, Johannisbrotgummi, Gellangummi und Carrageen, ausgewählt ist.

25. Zusammensetzung nach Anspruch 24,
wobei das gelbildende Polysaccharid ein Carrageen mit nicht mehr als 1,0 Sulfatresten pro Disaccharidrepetiereinheit ist.

26. Zusammensetzung nach Anspruch 24,
wobei das Carrageen Eucheuma-Carrageen oder Furcellaran ist.

27. Zusammensetzung nach Anspruch 21,
wobei die Viskosität vor der Verabreichung im Bereich von etwa 1 bis etwa 5000 mPa.s (cps) und die Viskosität nach der Verabreichung über etwa 50 mPa.s (cps) liegt.

28. Zusammensetzung nach Anspruch 21,
wobei die Viskosität vor der Verabreichung im Bereich von etwa 1 bis 500 mPa.s (cps) liegt und das Gel nach der Verabreichung ein Elastizitätsmodul im Bereich von 1 x 10⁴ bis etwa 5 x 10⁵ dPa (DYN/cm²) aufweist.

29. Zusammensetzung nach Anspruch 21,
wobei das Elastizitätsmodul vor der Verabreichung im Bereich von etwa 1 x 10⁴ bis etwa 3 x 10⁵ dPa (DYN/cm²) und das Elastizitätsmodul nach der Verabreichung im Bereich von etwa 1 x 10⁴ bis etwa 2 x 10⁶ dPa (DYN/cm²) liegt.

30. Zusammensetzung nach Anspruch 21,
wobei die Zusammensetzung thixotrop ist und ein Elastizitätsmodul im Bereich von etwa 1 x 10⁴ bis etwa 2 x 10⁵ dPa (DYN/cm²) und, nach dem Schütteln, eine Viskosität vor der Verabreichung im Bereich von etwa 1 bis etwa 5000 mPa.s (cps) und ein Elastizitätsmodul nach der Verabreichung im Bereich von etwa 1 x 10⁴ bis etwa 2 x 10⁶ dPa (DYN/cm₂) aufweist.

31. Verwendung einer ophthalmischen Zusammensetzung, die kein Arzneimittel enthält, umfassend ein gelbildendes Polysaccharid und ein fein zerteiltes Arzneimittelträgersubstrat, wobei die Konzentration des gelbildenden Polysaccharids derart ist, daß die Zusammensetzung als Tropfen verabreichbar ist und beim Eintropfen geliert, nach einem der Ansprüche 21 bis 30, zur Herstellung eines Arzneimittels, um das Auge zu lubrifizieren oder dem Auge Tränen zu supplementieren.

## Patentansprüche (Patentansprüche für folgende(n) Vertragsstaat(en): ES, GR)

1. Verfahren zur Herstellung einer topischen ophthalmischen Zusammensetzung mit verzögerter Freisetzung, umfassend das Vermischen eines Arzneimittels, eines gelbildenden Polysaccharids und eines fein zerteilten Arzneimittelträgersubstrats, wobei die Konzentration des gelbildenden Polysaccharids derart ist, daß die Zusammensetzung als Tropfen verabreichbar ist und beim Eintröpfeln geliert, so daß eine topische ophthalmische Zusammensetzung mit verzögerter Freisetzung ausgebildet wird.

2. Verfahren nach Anspruch 1,
wobei das fein zerteilte Arzneimittelträgersubstrat ein Ionenaustauschharz ist.

3. Verfahren nach Anspruch 1,
wobei die Konzentration des gelbildenden Polysaccharids zwischen etwa 0,1 % bis etwa 3,0 % w/v und die Konzentration des fein zerteilten Arzneimittelträgersubstrats zwischen etwa 0,05 % bis etwa 10,0 % w/v liegt.

4. Verfahren nach Anspruch 1,
wobei das gelbildende Polysaccharid aus der Gruppe, bestehend aus Xanthangummi, Johannisbrotgummi, Gellangummi und Carrageen ausgewählt ist.

5. Verfahren nach Anspruch 4,
wobei das gelbildende Polysaccharid ein Carrageen mit nicht mehr als 1,0 Sulfatresten pro Disaccharidrepetiereinheit ist.

6. Verfahren nach Anspruch 4,
wobei das Carrageen Eucheuma-Carrageen oder Furcellaran ist.

7. Verfahren nach Anspruch 1,
wobei die Viskosität vor der Verabreichung im Bereich von etwa 1 bis etwa 5000 mPa.s (cps) und die Viskosität nach der Verabreichung über etwa 50 mPa.s (cps) liegt.

8. Verfahren nach Anspruch 1,
wobei die Viskosität vor der Verabreichung im Bereich von etwa 1 bis etwa 500 mPa.s (cps) liegt und das Gel nach der Verabreichung ein Elastizitätsmodul im Bereich von 1 x 10⁴ bis etwa 5 x 10⁵ dPa (DYN/cm²) aufweist.

9. Verfahren nach Anspruch 1,
wobei das Elastizitätsmodul vor der Verabreichung im Bereich von etwa 1 x 10⁴ bis etwa 3 x 10⁵ dPa (DYN/cm²) und das Elastizitätsmodul nach der Verabreichung im Bereich von etwa 1 x 10⁴ bis etwa 2 x 10⁶ dPa (DYN/cm²) liegt.

10. Verfahren nach Anspruch 1,
wobei die Zusammensetzung thixotrop ist und ein Elastizitätsmodul im Bereich von etwa 1 x 10⁴ bis etwa 2 x 10⁵ dPa (DYN/cm²) und, nach dem Schütteln, eine Viskosität vor der Verabreichung im Bereich von etwa 1 bis etwa 5000 mPa.s (cps) und ein Elastizitätsmodul nach der Verabreichung im Bereich von etwa 1 x 10⁴ bis etwa 2 x 10⁶ dPa (DYN/cm₂) aufweist.

11. Verfahren nach Anspruch 1,
wobei das Arnzeimittel aus der Gruppe, bestehend aus Glaucomamitteln, Antihypertensiva, nicht steroidalen und steroidalen Entzündungshemmern, antibakteriellen Mitteln und antiinfektiven Mitteln, antifungativen Mitteln, antiviralen Mitteln, Antikataraktmitteln, Antioxidantien, Antiallergika, Wachstumsfaktoren und deren Vorläufern ausgewählt ist.

12. Verfahren nach Anspruch 11,
wobei das Glaucomamittel aus der Gruppe, bestehend aus β-Blockern, α-Agonisten, Carboanhydraseinhibitoren, Dopaminagonisten und -antagonisten, miotischen Cholinergika, Prostaglandinen, ACE-Inhibitoren, Steroiden und Calcium-Kanalblockern ausgewählt ist.

13. Verfahren nach Anspruch 11,
wobei das Arzneimittel aus der Gruppe, bestehend aus den racemischen und enantiomeren Formen der nachfolgenden Verbindungen ausgewählt ist: Betaxolol, Timolol, Carteolol, Apraclonidin, (+)-4-Ethylamino-2,3-dihydro-4-H-2-methylthieno[3,2-e]-1,2-thiazin-6-sulfonamid-1,1-dioxid, Pilocarpin, Carbachol, Prostaglandinen, ACE-Inhibitoren, Glucocorticoiden, angiostatischen Steroiden, Calcium-Kanalblockern, Diclofenac, Ketorolac, Fluormethalonacetat, Prednisolonacetat, Tetrahydrocortisol, Tobramycin, Chinolonen, Beta-Lactamen, Natamycin, Acyclovir und Ganciclovir.

14. Verwendung einer topischen ophthalmischen Zusammensetzung mit verzögerter Freisetzung zur Zufuhr eines Arzneimittels zu den Augen, umfassend eine Mischung aus einem Arzneimittel, einem gelbildenden Polysaccharid und einem fein zerteilten Arzneimittelträgersubstrat, wobei die Konzentration des gelbildenden Polysaccharids derart ist, daß die Zusammensetzung als Tropfen verabreichbar ist und beim Eintröpfeln geliert, zur Herstellung eines Arzneimittels zur topischen Zufuhr eines Arzneimittels zu den Augen.

15. Verwendung nach Anspruch 14,
wobei die Konzentration des gelbildenden Polysaccharids zwischen etwa 0,1 % bis etwa 3,0 % w/v und die Konzentration des fein zerteilten Arzneimittelträgersubstrats zwischen etwa 0,05 % bis etwa 10,0 % w/v liegt.

16. Verwendung nach Anspruch 15,
wobei das gelbildende Polysaccharid aus der Gruppe, bestehend aus Xanthangummi, Johannisbrotgummi, Gellangummi und Carrageen ausgewählt ist, und das fein zerteilte Arzneimittelträgersubstrat ein Ionenaustauschharz ist.

17. Verwendung nach Anspruch 16,
wobei das Carrageen Eucheuma-Carrageen oder Furcellaran ist und der Wirkstoff para-Aminoclonidin oder ein Carboanhydraseinhibitor ist.

18. Verwendung nach Anspruch 17,
wobei der Carboanhydraseinhibitor (+)-4-Ethylamino-2,2-dihydro-4-H-2-methylthieno[3,2-e]-1,2-thiazin-6-sulfonamid-1,1-dioxid oder ein pharmazeutisch verträgliches Salz dieser Verbindung ist.

19. Verfahren nach Anspruch 1,
wobei die fein zerteilte Arzneimittelträgersubstanz Amberlit-Harz ist und das gelbildende Polysaccharid Eucheuma ist.

20. Verwendung nach Anspruch 14,
wobei die fein zerteilte Arzneimittelträgersubstanz Amberlit-Harz ist und das gelbildende Polysaccharid Eucheuma ist.

21. Verfahren zur Herstellung einer kein Arzneimittel enthaltenden topischen ophthalmischen Zusammensetzung, umfassend das Vermischen eines gelbildenden Polysaccharids und eines fein zerteilten Arzneimittelträgersubstrats, wobei die Konzentration des gelbildenden Polysaccharids derart ist, daß die Zusammensetzung als Tropfen verabreichbar ist und beim Einträufeln geliert, zur Bildung einer topischen ophthalmischen Zusammensetzung ohne Arzneimittel.

22. Verfahren nach Anspruch 21,
wobei das fein zerteilte Arzneimittelträgersubstrat ein Ionenaustauschharz ist.

23. Verfahren nach Anspruch 21,
wobei die Konzentration des gelbildenden Polysaccharids zwischen etwa 0,1 % bis etwa 3,0 % w/v und die Konzentration des fein zerteilten Arzneimittelträgersubstrats zwischen etwa 0,05 % bis etwa 10,0 % w/v liegt.

24. Verfahren nach Anspruch 21,
wobei das gelbildende Polysaccharid aus der Gruppe, bestehend aus Xanthangummi, Johannisbrotgummi, Gellangummi und Carrageen, ausgewählt ist.

25. Verfahren nach Anspruch 24,
wobei das gelbildende Polysaccharid ein Carrageen mit nicht mehr als 1,0 Sulfatresten pro Disaccharidrepetiereinheit ist.

26. Verfahren nach Anspruch 24,
wobei das Carrageen Eucheuma-Carrageen oder Furcellaran ist.

27. Verfahren nach Anspruch 21,
wobei die Viskosität vor der Verabreichung im Bereich von etwa 1 bis etwa 5000 mPa.s (cps) und die Viskosität nach der Verabreichung über etwa 50 mPa.s (cps) liegt.

28. Verfahren nach Anspruch 21,
wobei die Viskosität vor der Verabreichung im Bereich von etwa 1 bis 500 mPa.s (cps) liegt und das Gel nach der Verabreichung ein Elastizitätsmodul im Bereich von 1 x 10⁴ bis etwa 5 x 10⁵ dPa (DYN/cm²) aufweist.

29. Verfahren nach Anspruch 21,
wobei das Elastizitätsmodul vor der Verabreichung im Bereich von etwa 1 x 10⁴ bis etwa 3 x 10⁵ dPa (DYN/cm²) und das Elastizitätsmodul nach der Verabreichung im Bereich von etwa 1 x 10⁴ bis etwa 2 x 10⁶ dPa (DYN/cm²) liegt.

30. Verfahren nach Anspruch 21,
wobei die Zusammensetzung thixotrop ist und ein Elastizitätsmodul im Bereich von etwa 1 x 10⁴ bis etwa 2 x 10⁵ dPa (DYN/cm²) und, nach dem Schütteln, eine Viskosität vor der Verabreichung im Bereich von etwa 1 bis etwa 5000 mPa.s (cps) und ein Elastizitätsmodul nach der Verabreichung im Bereich von etwa 1 x 10⁴ bis etwa 2 x 10⁶ dPa (DYN/cm₂) aufweist.

31. Verwendung einer ophthalmischen Zusammensetzung, die kein Arzneimittel enthält, umfassend ein gelbildendes Polysaccharid und ein fein zerteiltes Arzneimittelträgersubstrat, wobei die Konzentration des gelbildenden Polysaccharids derart ist, daß die Zusammensetzung als Tropfen verabreichbar ist und beim Eintropfen geliert, nach einem der Ansprüche 21 bis 30, zur Herstellung eines Arzneimittels, um das Auge zu lubrifizieren oder dem Auge Tränen zu supplementieren.

## Revendications (Revendications pour l'(les) Etat(s) contractant(s) suivant(s): AT, BE, CH, LI, DE, DK, FR, GB, IT, LU, MC, NL, PT, SE)

1. Composition oculaire topique à libération prolongée, comprenant un mélange d'un principe actif, d'un polysaccharide gélifiant et d'un substrat véhicule de principe actif finement divisé, où la concentration dudit polysaccharide gélifiant est telle que la composition est administrable sous la forme d'une goutte et de gels en instillation.

2. Composition selon la revendication 1, où le substrat véhicule de principe actif finement divisé est une résine échangeuse d'ions.

3. Composition selon la revendication 1, où la concentration du polysaccharide gélifiant est comprise entre environ 0,1 % et environ 3,0 % en poids/volume et la concentration du substrat véhicule de principe actif finement divisé est comprise entre environ 0,05 % et environ 10,0 % en poids/volume.

4. Composition selon la revendication 1, où le polysaccharide gélifiant est sélectionné dans le groupe constitué par : la gomme de xanthane, la gomme de caroube, la gomme de gellane et la carragénine.

5. Composition selon la revendication 4, où le polysaccharide gélifiant est une carragénine n'ayant pas plus de 1,0 fraction sulfate par motif disaccharidique récurrent.

6. Composition selon la revendication 4, où ladite carragénine est de la carragénine d'eucheuma ou de la furcellarane.

7. Composition selon la revendication 1, où la viscosité avant administration est comprise dans la plage d'environ 1 à environ 5 000 mPa.s (cP) et la viscosité après administration est supérieure à environ 50 mPa.s (cP).

8. Composition selon la revendication 1, où la viscosité avant administration est comprise dans la plage d'environ 1 à environ 500 mPa.s (cP) et le gel après administration a un module d'élasticité compris dans la plage de 1 x 10⁴ à environ 5 x 10⁵ dPa (dynes/cm²).

9. Composition selon la revendication 1, où le module d'élasticité avant administration est compris dans la plage d'environ 1 x 10⁴ à environ 3 x 10⁵ dPa (dynes/cm²) et le module d'élasticité après administration est compris dans la plage d'environ 1 x 10⁴ à environ 2 x 10⁶ dPa (dynes/cm²).

10. Composition selon la revendication 1, où la composition est thixotrope, ayant un module d'élasticité avant administration compris dans la plage d'environ 1 x 10⁴ à environ 2 x 10⁵ dPa (dynes/cm²) et, après agitation, une viscosité avant administration comprise dans la plage d'environ 1 à environ 5 000 mPa.s (cP) et ayant un module d'élasticité après administration compris dans la plage d'environ 1 x 10⁴ à environ 2 x 10⁶ dPa (dynes/cm²).

11. Composition selon la revendication 1, où le principe actif est sélectionné dans le groupe constitué par : des agents de traitement du glaucome, des anti-hypertenseurs, des anti-inflammatoires stéroïdiens et non stéroïdiens, des antibactériens et des anti-infectieux, des antifongiques, des antiviraux, des agents de traitement de la cataracte, des antioxydants, des antiallergiques, des facteurs de croissance et leurs précurseurs.

12. Composition selon la revendication 11, où l'agent de traitement du glaucome est sélectionné dans le groupe constitué par : les β-bloquants, les α-stimulants, les inhibiteurs de l'anhydrase carbonique, les agonistes et les antagonistes de la dopamine, les cholinergiques myotiques, les prostaglandines, les inhibiteurs de l'enzyme de conversion de l'angiotensine, les stéroïdiens et les anticalciques.

13. Composition selon la revendication 11, où le principe actif est sélectionné dans le groupe constitué par les formes racémiques et énantiomères de : bêtaxolol, timolol, cartéolol, apraclonidine, 1,1-dioxyde de (+)-4-éthylamino-2,3-dihydro-4-H-2-méthylthiéno[3,2-e]-1,2-thiazine-6-sulfonamide, pilocarpine, carbachol, prostaglandines, inhibiteurs de l'enzyme de conversion de l'angiotensine, glucocorticoïdes, stéroïdiens angiostatiques, anticalciques, diclofénac, kétorolac, acétate de fluorométhalone, acétate de prednisolone, tétrahydrocortisol, tobramycine, quinolones, bêta-lactames, natamycine, aciclovir et ganciclovir.

14. Utilisation d'une composition oculaire topique à libération prolongée pour l'apport d'un principe actif aux yeux, comprenant un mélange d'un principe actif, d'un polysaccharide gélifiant et d'un substrat véhicule de principe actif finement divisé, où la concentration dudit polysaccharide gélifiant est telle que la composition est administrable sous la forme d'une goutte et de gels en instillation, en vue de la préparation d'un médicament destiné à l'apport topique d'un principe actif aux yeux.

15. Utilisation selon la revendication 14, où la concentration du polysaccharide gélifiant est comprise entre environ 0,1 % et environ 3,0 % en poids/volume et la concentration du substrat véhicule de principe actif finement divisé est comprise entre environ 0,05 % et environ 10,0 % en poids.

16. Utilisation selon la revendication 15, où le polysaccharide gélifiant est sélectionné dans le groupe constitué par : la gomme de xanthane, la gomme de caroube, la gomme de gellane et la carragénine et le substrat véhicule de principe actif finement divisé est une résine échangeuse d'ions.

17. Utilisation selon la revendication 16, où ladite carragénine est de la carragénine d'eucheuma ou de la furcellarane et le médicament est de la para-aminoclonidine ou un inhibiteur de l'anhydrase carbonique.

18. Utilisation selon la revendication 17, où l'inhibiteur de l'anhydrase carbonique est le 1,1-dioxyde de (+)-4-éthylamino-2,2-dihydro-4-H-2-méthylthiéno[3,2-e]-1,2-thiazine-6-sulfonamide ou un sel pharmaceutiquement acceptable de ce dernier.

19. Composition selon la revendication 1, où la substance véhicule de principe actif finement divisé est de la résine Amberlite® et le polysaccharide gélifiant est eucheuma.

20. Utilisation selon la revendication 14, où la substance véhicule de principe actif finement divisé est la résine Amberlite® et le polysaccharide gélifiant est eucheuma.

21. Composition oculaire topique ne contenant pas de principe actif, comprenant un polysaccharide gélifiant et un substrat véhicule de principe actif finement divisé, où la concentration dudit polysaccharide gélifiant est telle que la composition est administrable sous forme d'une goutte et de gels en instillation.

22. Composition selon la revendication 21, où le substrat véhicule de principe actif finement divisé est une résine échangeuse d'ions.

23. Composition selon la revendication 21, où la concentration du polysaccharide gélifiant est comprise entre environ 0,1 % et environ 3,0 % en poids/volume et la concentration du substrat véhicule de principe actif finement divisé est comprise entre environ 0,05 % et environ 10,0 % en poids/volume.

24. Composition selon la revendication 21, où le polysaccharide gélifiant est sélectionné dans le groupe constitué par : la gomme de xanthane, la gomme de caroube, la gomme de gellane et la carragénine.

25. Composition selon la revendication 24, où le polysaccharide gélifiant est une carragénine n'ayant pas plus de 1,0 fraction sulfate par motif disaccharidique récurrent.

26. Composition selon la revendication 24, où ladite carragénine est de la carragénine d'eucheuma ou de la furcellarane.

27. Composition selon la revendication 21, où la viscosité avant administration est comprise dans la plage d'environ 1 à environ 5 000 mPa.s (cP) et la viscosité après administration est supérieure à environ 50 mPa.s (cP).

28. Composition selon la revendication 21, où la viscosité avant administration est comprise dans la plage d'environ 1 à environ 500 mPa.s (cP) et le gel après administration a un module d'élasticité compris dans la plage de 1 x 10⁴ à environ 5 x 10⁵ dPa (dynes/cm²).

29. Composition selon la revendication 21, où le module d'élasticité avant administration est compris dans la plage d'environ 1 x 10⁴ à environ 3 x 10⁵ dPa (dynes/cm²) et le module d'élasticité après administration est compris dans la plage d'environ 1 x 10⁴ à environ 2 x 10⁶ dPa (dynes/cm²).

30. Composition selon la revendication 21, où la composition est thixotrope, ayant un module d'élasticité avant administration compris dans la plage d'environ 1 x 10⁴ à environ 2 x 10⁵ dPa (dynes/cm²) et, après agitation, une viscosité avant administration comprise dans la plage d'environ 1 à environ 5 000 mPa.s (cP) et ayant un module d'élasticité après administration compris dans la plage d'environ 1 x 10⁴ à environ 2 x 10⁶ dPa (dynes/cm²).

31. Utilisation d'une composition oculaire ne contenant pas de principe actif, comprenant un polysaccharide gélifiant et un substrat véhicule de principe actif finement divisé, où la concentration dudit polysaccharide gélifiant est telle que la composition est administrable sous la forme d'une goutte et de gels en instillation selon l'une quelconque des revendications 21 à 30, en vue de la préparation d'un médicament destiné à lubrifier l'oeil ou à apporter des larmes à l'oeil.

## Revendications (Revendications pour l'(les) Etat(s) contractant(s) suivant(s): ES, GR)

1. Procédé de préparation d'une composition oculaire topique à libération prolongée, comprenant le mélange d'un principe actif, d'un polysaccharide gélifiant et d'un substrat véhicule de principe actif finement divisé, où la concentration dudit polysaccharide gélifiant est telle que la composition est administrable sous forme d'une goutte et de gels en instillation pour former une composition oculaire topique à libération prolongée.

2. Procédé selon la revendication 1, où le substrat véhicule de principe actif finement divisé est une résine échangeuse d'ions.

3. Procédé selon la revendication 1, où la concentration du polysaccharide gélifiant est comprise entre environ 0,1 % et environ 3,0 % en poids/volume et la concentration du substrat véhicule de principe actif finement divisé est comprise entre environ 0,05 % et environ 10,0 % en poids/volume.

4. Procédé selon la revendication 1, où le polysaccharide gélifiant est sélectionné dans le groupe constitué par : la gomme de xanthane, la gomme de caroube, la gomme de gellane et la carragénine.

5. Procédé selon la revendication 4, où le polysaccharide gélifiant est une carragénine n'ayant pas plus de 1,0 fraction sulfate par motif disaccharidique récurrent.

6. Procédé selon la revendication 4, où ladite carragénine est de la carragénine d'eucheuma ou de la furcellarane.

7. Procédé selon la revendication 1, où la viscosité avant administration est comprise dans la plage d'environ 1 à environ 5 000 mPa.s (cP) et la viscosité après administration est supérieure à environ 50 mPa.s (cP).

8. Procédé selon la revendication 1, où la viscosité avant administration est comprise dans la plage d'environ 1 à environ 500 mPa.s (cP) et le gel après administration a un module d'élasticité compris dans la plage de 1 x 10⁴ à environ 5 x 10⁵ dPa (dynes/cm²).

9. Procédé selon la revendication 1, où le module d'élasticité avant administration est compris dans la plage d'environ 1 x 10⁴ à environ 3 x 10⁵ dPa (dynes/cm²) et le module d'élasticité après administration est compris dans la plage d'environ 1 x 10⁴ à environ 2 x 10⁶ dPa (dynes/cm²).

10. Procédé selon la revendication 1, où la composition est thixotrope, ayant un module d'élasticité avant administration compris dans la plage d'environ 1 x 10⁴ à environ 2 x 10⁵ dPa (dynes/cm²) et, après agitation, une viscosité avant administration comprise dans la plage d'environ 1 à environ 5 000 mPa.s (cP) et ayant un module d'élasticité après administration compris dans la plage d'environ 1 x 10⁴ à environ 2 x 10⁶ dPa (dynes/cm²).

11. Procédé selon la revendication 1, où le principe actif est sélectionné dans le groupe constitué par : des agents de traitement du glaucome, des anti-hypertenseurs, des anti-inflammatoires stéroïdiens et non stéroïdiens, des antibactériens et des anti-infectieux, des antifongiques, des antiviraux, des agents de traitement de la cataracte, des antioxydants, des antiallergiques, des facteurs de croissance et leurs précurseurs.

12. Procédé selon la revendication 11, où l'agent de traitement du glaucome est sélectionné dans le groupe constitué par : les β-bloquants, les α-stimulants, les inhibiteurs de l'anhydrase carbonique, les agonistes et les antagonistes de la dopamine, les cholinergiques myotiques, les prostaglandines, les inhibiteurs de l'enzyme de conversion de l'angiotensine, les stéroïdiens et les anticalciques.

13. Procédé selon la revendication 11, où le principe actif est sélectionné dans le groupe constitué par les formes racémiques et énantiomères de : bêtaxolol, timolol, cartéolol, apraclonidine, 1,1-dioxyde de (+)-4-éthylamino-2,3-dihydro-4-H-2-méthylthiéno[3,2-e]-1,2-thiazine-6-sulfonamide, pilocarpine, carbachol, prostaglandines, inhibiteurs de l'enzyme de conversion de l'angiotensine, glucocorticoïdes, stéroïdiens angiostatiques, anticalciques, diclofénac, kétorolac, acétate de fluorométhalone, acétate de prednisolone, tétrahydrocortisol, tobramycine, quinolones, bêta-lactames, natamycine, aciclovir et ganciclovir.

14. Utilisation d'une composition oculaire topique à libération prolongée pour l'apport d'un principe actif aux yeux, comprenant un mélange d'un principe actif, d'un polysaccharide gélifiant et d'un substrat véhicule de principe actif finement divisé, où la concentration dudit polysaccharide gélifiant est telle que la composition est administrable sous la forme d'une goutte et de gels en instillation, en vue de la préparation d'un médicament destiné à l'apport topique d'un principe actif aux yeux.

15. Utilisation selon la revendication 14, où la concentration du polysaccharide gélifiant est comprise entre environ 0,1 % et environ 3,0 % en poids/volume et la concentration du substrat véhicule de principe actif finement divisé est comprise entre environ 0,05 % et environ 10,0 % en poids.

16. Utilisation selon la revendication 15, où le polysaccharide gélifiant est sélectionné dans le groupe constitué par : la gomme de xanthane, la gomme de caroube, la gomme de gellane et la carragénine et le substrat véhicule de principe actif finement divisé est une résine échangeuse d'ions.

17. Utilisation selon la revendication 16, où ladite carragénine est de la carragénine d'eucheuma ou de la furcellarane et le médicament est de la para-aminoclonidine ou un inhibiteur de l'anhydrase carbonique.

18. Utilisation selon la revendication 17, où l'inhibiteur de l'anhydrase carbonique est le 1,1-dioxyde de (+)-4-éthylamino-2,2-dihydro-4-H-2-méthylthiéno[3,2-e]-1,2-thiazine-6-sulfonamide ou un sel pharmaceutiquement acceptable de ce dernier.

19. Procédé selon la revendication 1, où la substance véhicule de principe actif finement divisé est de la résine Amberlite® et le polysaccharide gélifiant est eucheuma.

20. Utilisation selon la revendication 14, où la substance véhicule de principe actif finement divisé est la résine Amberlite® et le polysaccharide gélifiant est eucheuma.

21. Procédé de préparation d'une composition oculaire topique ne contenant pas de principe actif, comprenant le mélange d'un polysaccharide gélifiant et d'un substrat véhicule de principe actif finement divisé, où la concentration dudit polysaccharide gélifiant est telle que la composition est administrable sous la forme d'une goutte et de gels en instillation, pour former une composition oculaire topique ne contenant pas de principe actif.

22. Procédé selon la revendication 21, où le substrat véhicule de principe actif finement divisé est une résine échangeuse d'ions.

23. Procédé selon la revendication 21, où la concentration du polysaccharide gélifiant est comprise entre environ 0,1 % et environ 3,0 % en poids/volume et la concentration du substrat véhicule de principe actif finement divisé est comprise entre environ 0,05 % et environ 10,0 % en poids/volume.

24. Procédé selon la revendication 21, où le polysaccharide gélifiant est sélectionné dans le groupe constitué par : la gomme de xanthane, la gomme de caroube, la gomme de gellane et la carragénine.

25. Procédé selon la revendication 24, où le polysaccharide gélifiant est une carragénine n'ayant pas plus de 1,0 fraction sulfate par motif disaccharidique récurrent.

26. Procédé selon la revendication 24, où ladite carragénine est de la carragénine d'eucheuma ou de la furcellarane.

27. Procédé selon la revendication 21, où la viscosité avant administration est comprise dans la plage d'environ 1 à environ 5 000 mPa.s (cP) et la viscosité après administration est supérieure à environ 50 mPa.s (cP).

28. Procédé selon la revendication 21, où la viscosité avant administration est comprise dans la plage d'environ 1 à environ 500 mPa.s (cP) et le gel après administration a un module d'élasticité compris dans la plage de 1 x 10⁴ à environ 5 x 10⁵ dPa (dynes/cm²).

29. Procédé selon la revendication 21, où le module d'élasticité avant administration est compris dans la plage d'environ 1 x 10⁴ à environ 3 x 10⁵ dPa (dynes/cm²) et le module d'élasticité après administration est compris dans la plage d'environ 1 x 10⁴ à environ 2 x 10⁶ dPa (dynes/cm²).

30. Procédé selon la revendication 21, où la composition est thixotrope, ayant un module d'élasticité avant administration compris dans la plage d'environ 1 x 10⁴ à environ 2 x 10⁵ dPa (dynes/cm²) et, après agitation, une viscosité avant administration comprise dans la plage d'environ 1 à environ 5 000 mPa.s (cP) et ayant un module d'élasticité après administration compris dans la plage d'environ 1 x 10⁴ à environ 2 x 10⁶ dPa (dynes/cm²).

31. Utilisation d'une composition oculaire ne contenant pas de principe actif, comprenant un polysaccharide gélifiant et un substrat véhicule de principe actif finement divisé, où la concentration dudit polysaccharide gélifiant est telle que la composition est administrable sous la forme d'une goutte et de gels en instillation selon l'une quelconque des revendications 21 à 30, en vue de la préparation d'un médicament destiné à lubrifier l'oeil ou à apporter des larmes à l'oeil.
